(19) Europäisches Patentamt
European Patent Office
Office européen des brevets

(11) EP 1 446 100 B1

(12) EUROPEAN PATENT SPECIFICATION

(45) Date of publication and mention of the grant of the patent:
**04.05.2011 Bulletin 2011/18**

(21) Application number: **02793941.2**

(22) Date of filing: **14.11.2002**

(51) Int Cl.:
***A61K 9/00*** *(2006.01)*

(86) International application number:
**PCT/US2002/036715**

(87) International publication number:
**WO 2003/041684 (22.05.2003 Gazette 2003/21)**

(54) **INJECTABLE DEPOT COMPOSITIONS AND USES THEREOF**

INJIZIERBARE DEPOTZUSAMMENSETZUNGEN UND DEREN VERWENDUNG

COMPOSITIONS EN DEPOT INJECTABLES ET UTILISATIONS

(84) Designated Contracting States:
**AT BE BG CH CY CZ DE DK EE ES FI FR GB GR IE IT LI LU MC NL PT SE SK TR**

(30) Priority: **14.11.2001 US 336307 P**

(43) Date of publication of application:
**18.08.2004 Bulletin 2004/34**

(73) Proprietor: **Durect Corporation
Cupertino, CA 95014 (US)**

(72) Inventors:
- **CHEN, Guohua
Sunnyvale, CA 94086 (US)**
- **HOUSTON, Paul, Ricky
Hayward, CA 94542 (US)**
- **KLEINER, Lothar, Walther
Los Altos, CA 94022 (US)**
- **WRIGHT, Jeremy, Corwin
Los Altos, CA 94024-4135 (US)**

(74) Representative: **Bradley, Adrian et al
Cleveland
40-43 Chancery Lane
London WC2A 1JQ (GB)**

(56) References cited:
**WO-A-00/74650**
**WO-A-93/24150**
**WO-A-95/13799**
**WO-A-98/27963**
**WO-A-02/067991**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 446 100 B1

Printed by Jouve, 75001 PARIS (FR)

## Description

BACKGROUND OF THE INVENTION

### Technical Field

The present invention relates to a depot composition that can be injected into a desired location within a patient's body to form an implant, which provides for sustained release of a beneficial agent, for use in a method to administer a beneficial agent to a patient.

### Background

Biodegradable polymers have been used for many years in medical applications. Illustrative devices composed of the biodegradable polymers include sutures, surgical clips, staples, implants, and drug delivery systems. The majority of these biodegradable polymers have been based upon glycolide, lactide, caprolactone, and copolymers thereof.

The biodegradable polymers can be thermoplastic materials, meaning that they can be heated and formed into various shapes such as fibers, clips, staples, pins, films, etc. Alternatively, they can be thermosetting materials formed by crosslinking reactions, which lead to high-molecular-weight materials that do not melt or form flowable liquids at high temperatures.

Although thermoplastic and thermosetting biodegradable polymers have many useful biomedical applications, there are several important limitations to their use in the bodies of various animals including humans, animals, birds, fish, and reptiles. Because these polymers are solids, all instances involving their use have required initially forming the polymeric structures outside the body, followed by insertion of the solid structure into the body. For example, sutures, clips, and staples are all formed from thermoplastic biodegradable polymers prior to use. When inserted into the body, they retain their original shape. While this characteristic is essential for some uses, it is a drawback where it is desired that the material flow to fill voids or cavities where it may be most needed.

Drug delivery systems using thermoplastic or thermosetting biodegradable polymers also have to be formed outside the body. In such instances, the drug is incorporated into the polymer and the mixture is shaped into a certain form such as a cylinder, disc, or fiber for implantation. With such solid implants, the drug delivery system has to be inserted into the body through an incision. These incisions are sometimes larger than desired by the medical profession and occasionally lead to a reluctance of the patients to accept such an implant or drug delivery system. Nonetheless, both biodegradable and non-biodegradable implantable drug delivery systems have been widely used successfully.

One reservoir device having a rate-controlling membrane and zero-order release of an agent that is particularly designed for intraoral implantation is described in U.S. Patent No. 5,085,866. The device is prepared from a core that is sprayed with a solution having a polymer and a solvent that is composed of a rapidly evaporating, low boiling point first solvent and a slowly evaporating, high boiling second solvent.

Other illustrative osmotic delivery systems include those disclosed in U.S. Patent Nos. 3,797,492, 3,987,790, 4,008,719, 4,865,845, 5,057,318, 5,059,423, 5,112,614, 5,137,727, 5,151,093, 5,234,692, 5,234,693, 5,279,608, and 5,336,057. Pulsatile delivery devices are also known which deliver a beneficial agent in a pulsatile manner as disclosed in U.S. Patent Nos. 5,209,746, 5,308,348, and 5,456,679.

One way to avoid the incision needed to implant drug delivery systems is to inject them as small particles, microspheres, or microcapsules. For example, U.S. Patent No. 5,019,400 describes the preparation of controlled release microspheres via a very low temperature casting process. These materials may or may not contain a drug that can be released into the body. Although these materials can be injected into the body with a syringe, they do not always satisfy the demand for a biodegradable implant. Because they are particulate in nature, they do not form a continuous film or solid implant with the structural integrity needed for certain prostheses. When inserted into certain body cavities such as a mouth, a periodontal pocket, the eye, or the vagina where there is considerable fluid flow, these small particles, microspheres, or microcapsules are poorly retained because of their small size and discontinuous nature. Further, the particles tend to aggregate and thus their behavior is hard to predict. In addition, microspheres or microcapsules prepared from these polymers and containing drugs for release into the body are sometimes difficult to produce on a large scale, and their storage and injection characteristics present problems. Furthermore, one other major limitation of the microcapsule or small-particle system is their lack of reversibility without extensive surgical intervention. That is, if there are complications after they have been injected, it is considerably more difficult to remove them from the body than with solid implants. A still further limitation on microparticles or microcapsulation is the difficulty in encapsulating protein and DNA-based drugs without degradation caused by solvents and temperature extremes.

The art has developed various drug delivery systems in response to the aforementioned challenges. For instance, U.S. Patent No. 4,938,763 and its divisional U.S. Patent No. 5,278,201 relate to a biodegradable polymer for use in providing syringeable, in-situ forming, solid biodegradable implants for animals. In one embodiment, a thermoplastic

system is used wherein a non-reactive polymer is dissolved in a biocompatible solvent to form a liquid which is placed in the animal wherein the solvent dissipates to produce the solid implant. Alternatively, a thermosetting system is used wherein effective amounts of a liquid acrylic ester-terminated, biodegradable prepolymer and a curing agent are formed and the liquid mixture is placed within the animal wherein the prepolymer cures to form the solid implant. It is stated that the systems provide a syringeable, solid biodegradable delivery system by the addition of an effective level of a biologically active agent to the liquid before the injection into the animal.

U.S. Patent 5,599,552 describes thermoplastic and thermoset polymer compositions that utilize solvents that are miscible to dispersible in water, such as N-methyl-2-pyrrolidone, resulting in polymer solutions capable of quickly absorbing water from surrounding tissue. The polarity of the solvents is described as being effective to provide about at least 10% solubility in water. The polymer matrix systems are described as forming a porous core surrounded by a porous skin.

U.S. Patent No. 5,242,910 describes a sustained release composition for treating periodontal disease. The composition comprises copolymers of lactide and glycolide, triacetin (as a solvent/plasticizer) and an agent providing relief of oral cavity diseases. The composition can take the form of a gel and can be inserted into a periodontal cavity via a syringe using either a needle or a catheter. As additional optional components, the composition can contain surfactants, flavoring agents, viscosity controlling agents, complexing agents, antioxidants, other polymers, gums, waxes/ oils, and coloring agents. One illustrative viscosity controlling agent set forth in one of the examples is polyethylene glycol 400. U.S. Patent Nos. 5,620,700 and 5,556,905 relate to polymer compositions for injectable implants using solvents and/or plasticizers.

Prior art polymer compositions for injectable implants have used solvent/plasticizers that are very or relatively soluble in aqueous body fluids to promote rapid solidification of the polymer at the implant site and promote diffusion of drug from the implant. However, it has now been observed that a serious problem associated with prior art polymeric implants utilizing water soluble polymer solvents is the rapid migration of water into the polymer composition when the implant is placed in the body and exposed to aqueous body fluids. That characteristic often results in uncontrolled release of beneficial agent that is manifested by an initial, rapid release of beneficial agent from the polymer composition, corresponding to a "burst" of beneficial agent being released from the implant. The burst often results in a substantial portion of the beneficial agent, if not all, being released in a very short time, e.g., hours or 1-2 days. Such an effect can be unacceptable, particularly in those circumstances where sustained delivery is desired, i.e., delivery of beneficial agent over a period of a week or a month or more, or where there is a narrow therapeutic window and release of excess beneficial agent can result in adverse consequences to the subject being treated, or where it is necessary to mimic the naturally-occurring daily profile of beneficial agents, such as hormones and the like, in the body of the subject being treated.

In an attempt to control burst and modulate and stabilize the delivery of the beneficial agent the prior art has coated particles of beneficial agent to retard release into an aqueous environment and extend release of the beneficial agent over time. Alternatively, various stabilizing or release modulating agents, such as metal salts as described in U.S. Patents 5,656,297, 5,654,010, 4,985,404 and 4,853,218 have been used. U.S. Patent No. 3,923,939 describes a method of reducing initial burst of an active agent from a delivery device by removing, prior to implantation, active agent from the exterior surface of the delivery device and through a layer of at least 5% of the overall body thickness extending from the exterior surface of the device.

Notwithstanding some success, those methods have not been entirely satisfactory for the large number of beneficial agents that would be effectively delivered by implants, since in many instances the modulation and stabilization effect is the result of the formation of a complex of the metal ion with the beneficial agent. When such complexes do not form, the stabilization/ modulation effect may not be adequate to prevent undesirable "burst" of the beneficial agent upon its introduction into the implant site.

The rapid water uptake into the polymer implant and solvent dispersion into body fluids exhibited by prior art devices often results in implants having pore structures that are non-homogeneous in size and shape. Typically, the surface pores take on a finger-like pore structure extending for as much as one-third of a millimeter or more from the implant surface into the implant, and such finger-like pores are open at the surface of the implant to the environment of use. The internal pores tend to be smaller and less accessible to the fluids present in the environment of use. Accordingly, when such devices are implanted, the finger-like pores allow very rapid uptake of aqueous body fluids into the interior of the implant with consequent immediate and rapid dissolution of significant quantities of beneficial agent and unimpeded diffusion of beneficial agent into the environment of use, producing the burst effect discussed above.

Furthermore, rapid water uptake can result in premature polymer precipitation such that a hardened implant or one with a hardened skin is produced. The inner pores and much of the interior of the polymer containing beneficial agent are shut off from contact with the body fluids and a significant reduction in the release of beneficial agent can result over a not insignificant period of time ("lag time"). That lag time is undesirable from the standpoint of presenting a controlled, sustained release of beneficial agent to the subject being treated. What one observes, then, is a burst of beneficial agent being released in a short time period immediately after implantation, a lag time in which no or very little beneficial agent is being released, and subsequently continued delivery of beneficial agent (assuming beneficial agent

remains after the burst) until the supply of beneficial agent is exhausted.

With solvent-based depot compositions comprised of a polymer dissolved in a solvent, the composition solidifies after injection as solvent diffuses from the depot. Since these compositions need to be non-viscous in order to be injected, a large percentage of drug is released as the system forms by diffusion of the solvent. This effect is referred to as a "burst" effect. In this respect, it is typical for solvent-based compositions to have a drug burst wherein 30-75% of the drug contained in the composition is released within one day of the initial injection.

An additional problem encountered with prior solvent-based depot compositions is that the viscosity of the injectable composition is relatively high, particularly when higher molecular weight polymers are used, and the injection force needed to introduce the composition into a patient's body is therefore high as well (*see*, e.g. U.S. Patent No. 6,130,200, WO 98/27963 A and WO 00/74650 A. To address this problem, those working in the field have employed lower molecular weight polymers and relatively volatile, water-soluble solvents such as ethanol. See, for example, U.S. Patent No. 5,733,950, 5,780,044, and 5,990,194 to Dunn et al and PCT publication WO 98/27962. However, these approaches can result in drug particle settling and/or a higher initial release burst and/or relatively large amounts of emulsifying agent, e.g., about one-third of the total weight of the composition. Furthermore, solvent volatility is problematic from a manufacturing standpoint, and monohydric lower alkanols such as ethanol can denature proteins and peptide drugs. Additionally, the requirement that the bioerodible polymer have a low molecular weight is quite restrictive from a manufacturing standpoint.

## SUMMARY OF THE INVENTION

The present invention is directed to the aforementioned needs in the art, and provides an injectable depot composition that exhibits improved shear thinning behavior and thereby enables reduced injection force and use of a small diameter (e.g., 16 gauge and higher) needle. The composition provides sustained release of a beneficial agent while limiting any initial burst effect, and offers increased formulation flexibility with regard to the polymer/solvent ratio and the molecular weight of the bioerodible polymer. Furthermore, the present composition does not contain volatile and/or potentially denaturing solvents such as ethanol.

In one aspect, then, the invention is directed to a composition comprising: a bioerodible, biocompatible polymer in an amount of from 35 wt. % to 75 wt. % of the composition; an aromatic alcohol having miscibility in water of less than or equal to 7 wt. % at 25 °C, in an amount effective to plasticize the polymer and form a gel therewith; and a beneficial agent in the form of particles, wherein the composition is free of monohydric C1-C6 alkanols for use in a method of treatment by injection into a patient's body to form an implant.

Preferred compositions are not only free of monohydric lower alcohols, but are also free of solvents having miscibility in water that is greater than 7 wt.% at 25°C.

In another aspect, the invention pertains to an injectable depot composition as described above, wherein the viscous gel further comprises a polymer selected from the group consisting of polylactides, polyglycolides, poly(caprolactone), polyanhydrides, polyamines, polyesteramides, polyorthoesters, polydioxanones, polyacetals, polyketals, polycarbonates, polyphosphoesters, polyorthocarbonates, polyphosphazenes, succinates, poly(malic acid), poly(amino acids), polyvinylpyrrolidone, polyethylene glycol, polyhydroxycellulose, polyphosphoesters, polysaccharides, chitin, chitosan, hyaluronic acid, and copolymers, terpolymers and mixtures thereof. In preferred embodiments, the polymer is a lactic aid based polymer. Preferably, the polylactic acid polymer may have a weight average molecular weight in the range of about 1,000 to about 120,000; preferably about 5,000 to about 50,000; and more preferably about 8,000 to about 30,000.

In preferred embodiments, the solvent is selected from the aromatic alcohol, lower alkyl and aralkyl esters of aryl acids; aryl, aralkyl and lower alkyl ketones; and lower alkyl esters of citric acid. Preferably, the solvent is selected from benzyl alcohol, benzyl benzoate and ethyl benzoate. In preferred embodiments, the composition is free of solvents having miscibility in water that is greater than 7 wt.% at 25°C. Preferably the solvent has miscibility in water of less than 7 wt.%, more preferably less than 5 wt%, and more preferably less than 3 wt%.

In another aspect, the invention pertains to an injectable depot composition for use in a method of administering such composition as described above, wherein the beneficial agent is selected from a drug, proteins, enzymes, hormones, polynucleotides, nucleoproteins, polysaccharides, glycoproteins, lipoproteins, polypeptides, steroids, analgesics, local anesthetics, antibiotic agents, chemotherapeutic agents, immunosuppressive agents, anti-inflammatory agents, antiproliferative agents, antimitotic agents, angiogenic agents, anticoagulants, fibrinolytic agents, growth factors, antibodies, ocular drugs, and metabolites, analogs, derivatives, fragments, and purified, isolated, recombinant and chemically synthesized versions of these species. In preferred embodiments, the beneficial agent is human growth hormone, methionine-human growth hormone; des-phenylalanine human growth hormone, alpha-, beta- or gamma-interferon, erythropoietin, glugacon, calcitonin, heparin, interleukin-1, interleukin-2, Factor VIII, Factor IX, luteinizing hormone, relaxin, follicle-stimulating hormone, atrial natriuretic factor, filgrastim epidermal growth factors (EGFs), platelet-derived growth factor (PDGFs), insulin-like growth factors (IGFs), fibroblast-growth factors (FGFs), transforming-growth factors (TGFs), interleukins (ILs), colony-stimulating factors (CSFs, MCFs, GCSFs, GMCSFs), Interferons (IFNs), endothelial growth factors (VEGF, EGFs), erythropoietins (EPOs), angiopoietins (ANGs), placenta-derived growth factors (PIGFs), and hypoxia induced transcriptional regulators (HIFs). Preferably, the beneficial agent is present in an amount of from 0.1 to 50% by weight of the combined amounts of the polymer, the solvent and the beneficial agent. In preferred embodiments, the beneficial agent is in the form of particles dispersed or dissolved in the viscous gel, wherein the beneficial agent is in the form of particles having an average particle size of from 0.1 to 250 microns. In certain preferred embodiments, the beneficial agent is in the form of particles wherein the particle further comprises a component selected from the group consisting of a stabilizing agent, bulking agent, chelating agent and a buffering agent.

## BRIEF DESCRIPTION OF THE DRAWINGS

The foregoing and other objects, features and advantages of the present invention will be more readily understood upon reading the following detailed description in conjunction with the drawings in which:

Figure 1 is a graph illustrating the rheological behavior of depot vehicles formulated with different solvents, i.e., Formulations 5, 6 and 7.

Figure 2 is a graph illustrating the injection force required to dispense the Formulations 5, 6 and 7 from a 24-gauge needle at 1 ml/minute, at room temperature.

Figure 3 is a graph illustrating the injection force required to dispense injectable depot compositions formulated with varying poly(lactide-co-glycolide) weight average molecular weights in combination with benzyl benzoate or benzyl alcohol from a 24 gauge needle at 1 ml/minute, at room temperature.

Figure 4 is a graph illustrating the injection force required to dispense depot compositions formulated with varying poly(lactide-co-glycolide) weight average molecular weights in combination with benzyl benzoate or benzyl alcohol or mixtures thereof from a 24 gauge needle at 1 ml/minute, at room temperature.

Figure 5 is a graph illustrating the in vivo release profile of human growth hormone obtained from various depot formulations, including those of the present invention (Formulations 8-10).

Figure 6 is a graph illustrating the in vivo release profile of human growth hormone obtained from various depot formulations (Formulations 10 and 11).

Figure 7 is a graph illustrating the in vivo release profile of bupivacaine obtained from various depot formulations, including those of the present invention (Formulations 12 and 13).

Figure 8 is a graph illustrating the in vivo release profile of bupivacaine obtained from various depot formulations (Formulations 13 and 14).

Figure 9 is a graph illustrating the in vivo release profile of bupivacaine obtained from depot formulations, including those of the present invention (Formulations 15 and 16).

Figure 10 illustrates the stability of hGH in the various depot formulations, including those of the present invention, as a function of time at 5 °C.

Figure 11 illustrates the injection force of various depot formulations, including those of the present invention (Formulations 8-10 and 17).

Figure 12 illustrates the stability of PDGF in the various depot formulations, including those of the present invention, as a function of time at 5 °C (Formulations 36-39).

Figure 13 illustrates the stability of PDGF in the various depot formulations, including those of the present invention, as a function of time at 25 °C (Formulations 36-39).

Figure 14 illustrates the stability of PDGF in the various depot formulations, including those of the present invention, as a function of time at 40 °C (Formulations 36-39).

Figure 15 is a graph illustrating the in vivo release profile of PDGF obtained from various depot compositions, including those of the present invention (Formulations 36-39).

## DETAILED DESCRIPTION OF THE INVENTION

### Overview and Definitions:

The present invention is directed to an injectable depot composition that serves as an implanted sustained release beneficial agent delivery system after injection into a patient's body. The composition is a gel formed from a bioerodible, biocompatible polymer and an aromatic alcohol that has miscibility in water of less than or equal to 7% at 25°C, preferably less than or equal to 5% at 25°C. The aromatic alcohol may be present in combination with an aromatic acid ester, an aromatic ketone, or both.

The composition provides sustained release of the beneficial agent by restricting water migration from the aqueous environment surrounding the implant system, thus delivering the beneficial agent over a prolonged period of time. Water uptake is controlled by virtue of the water-immiscible aromatic alcohol. Because the polymer of the composition is bioerodible, the implant system does not have to be surgically removed after beneficial agent is depleted from the implant.

Generally, the compositions of the invention are gel-like and form with a substantially homogeneous non-porous structure throughout the implant upon implantation and during drug delivery, even as it hardens. Furthermore, while the polymer gel implant will slowly harden when subjected to an aqueous environment, the hardened implant may maintain a rubbery (non-rigid) composition with the glass transition temperature Tg being below 37°C.

Because the aromatic alcohol in these compositions itself acts as a thixotropic agent and thus substantially increases shear thinning as well as composition homogeneity, it is not normally necessary to introduce additional thixotropic agents. In some embodiments, however, shear thinning and/or homogeneity may be further improved (thereby improving release characteristics) by incorporating added thixotropic agents. Also, pore formers and solubility modulators of the beneficial agent may be added to the implant systems to provide desired release profiles from the implant systems, along with typical pharmaceutical excipients and other additives that do not change the beneficial aspects of the present invention.

The preferred compositions herein allow beneficial agent to be loaded into the interior of the polymer at levels that are above that required to saturate the beneficial agent in water, thereby facilitating zero order release of beneficial agent. Additionally, the preferred compositions may provide viscous gels that have a glass transition temperature that is less than 37°C, such that the gel remains non-rigid for a period of time after implantation of 24 hours or more.

In describing and claiming the present invention, the following terminology will be used in accordance with the definitions set out below.

The singular forms "a," "an" and "the" include plural referents unless the context clearly dictates otherwise. Thus, for example, reference to "a solvent" includes a single solvent as well as a mixture of two or more different solvents, reference to "a beneficial agent" includes a single beneficial agent as well as two or more different beneficial agents in combination, reference to "an aromatic alcohol" includes a single aromatic alcohol as well as a mixture of two or more different aromatic alcohols, and the like.

The term "beneficial agent" means an agent that effects a desired beneficial, often pharmacological, effect upon administration to a human or an animal, whether alone or in combination with other pharmaceutical excipients or inert ingredients.

As used herein, the term "polynucleotide" refers to a polymeric form of nucleotides of any length, either ribonucleotides or deoxyribonucleotides, and includes double- and single-stranded DNA and RNA. It also includes known types of modifications, substitutions, and internucleotide modifications, which are known in the art.

As used herein, the term "recombinant polynucleotide" refers to a polynucleotide of genomic, cDNA, semisynthetic, or synthetic origin which, by virtue of its origin or manipulation: is not associated with all or a portion of a polynucleotide with which it is associated in nature; is linked to a polynucleotide other than that to which it is linked in nature; or does not occur in nature.

As used herein, the term "polypeptide" refers to a polymer of amino acids, inlcuding for example, peptides, oligopeptides, and proteins and derivatives, analogs and fragments thereof, as well as other modifications known in the art, both naturally occurring and non-naturally occurring.

As used herein, the term "purified" and "isolated" when referring to a polypeptide or nucleotide sequence means that the indicated molecule is present in the substantial absence of other biological macromolecules of the same type. The term "purified" as used herein preferably means at least 75% by weight, more preferably at least 85% by weight, more preferably still at least 95% by weight, and most preferably at least 98% by weight, of biological macromolecules of the same type present.

The term "AUC" means the area under the curve obtained from an in vivo assay in a subject by plotting blood plasma concentration of the beneficial agent in the subject against time, as measured from the time of implantation of the composition, to a time "t" after implantation. The time t will correspond to the delivery period of beneficial agent to a subject.

The term "burst index" means, with respect to a particular composition intended for systemic delivery of a beneficial agent, the quotient formed by dividing (i) the AUC calculated for the first time period after implantation of the composition into a subject divided by the number of hours in the first time period ($t_1$), by (ii) the AUC calculated for the time period of delivery of beneficial agent, divided by the number of hours in the total duration of the delivery period ($t_2$). For example the burst index at 24 hours is the quotient formed by dividing (i) the AUC calculated for the first twenty-four hours after implantation of the composition into a subject divided by the number 24, by (ii) the AUC calculated for the time period of delivery of beneficial agent, divided by the number of hours in the total duration of the delivery period.

The phrase "dissolved or dispersed" is intended to encompass all means of establishing a presence of beneficial agent in the gel composition and includes dissolution, dispersion, suspension and the like.

The term "systemic" means, with respect to delivery or administration of a beneficial agent to a subject, that the beneficial agent is detectable at a biologically-significant level in the blood plasma of the subject.

The term "local" means, with respect to delivery or administration of a beneficial agent to a subject, that the

beneficial agent is delivered to a localized site in the subject but is not detectable at a biologically significant level in the blood plasma of the subject.

The term "gel vehicle" means the composition formed by mixture of the polymer and solvent in the absence of the beneficial agent.

The term "prolonged period" means a period of time over which release of a beneficial agent from the implant of the invention occurs, which will generally be about one week or longer, and preferably about 30 days or longer.

The term "initial burst" means, with respect to a particular composition of this invention, the quotient obtained by dividing (i) the amount by weight of beneficial agent released from the composition in a predetermined initial period of time after implantation, by (ii) the total amount of beneficial agent that is to be delivered from an implanted composition. It is understood that the initial burst may vary depending on the shape and surface area of the implant. Accordingly, the percentages and burst indices associated with initial burst described herein are intended to apply to compositions tested in a form resulting from dispensing of the composition from a standard syringe.

The term "solubility modulator" means, with respect to the beneficial agent, an agent that will alter the solubility of the beneficial agent, with reference to polymer solvent or water, from the solubility of beneficial agent in the absence of the modulator. The modulator may enhance or retard the solubility of the beneficial agent in the solvent or water. However, in the case of beneficial agents that are highly water soluble, the solubility modulator will generally be an agent that will retard the solubility of the beneficial agent in water. The effects of solubility modulators of the beneficial agent may result from interaction of the solubility modulator with the solvent, or with the beneficial agent itself, such as by the formation of complexes, or with both. For the purposes hereof, when the solubility modulator is "associated" with the beneficial agent, all such interactions or formations as may occur are intended. Solubility modulators may be mixed with the beneficial agent prior to its combination with the viscous gel or may be added to the viscous gel prior to the addition of the beneficial agent, as appropriate.

The terms "subject" and "patient" mean, with respect to the administration of a composition of the invention, an animal or a human being.

Since all solvents, at least on a molecular level, will be soluble in water (i.e., miscible with water) to some very limited extent, the term "immiscible" as used herein means that 7% or less by weight, preferably 5% or less, of the solvent is soluble in or miscible with water. For the purposes of this disclosure, solubility values of solvent in water are considered to be determined at 25°C. Since it is generally recognized that solubility values as reported may not always be conducted at the same conditions, solubility limits recited herein as percent by weight miscible or soluble with water as part of a range or upper limit may not be absolute. For example, if the upper limit on solvent solubility in water is recited herein as "7% by weight," and no further limitations on the solvent are provided, the solvent "triacetin," which has a reported solubility in water of 7.17 grams in 100 ml of water, is considered to be included within the limit of 7%. A solubility limit in water of less than 7% by weight as used herein does not include the solvent triacetin or solvents having solubilities in water equal to or greater than triacetin.

The term "bioerodible" refers to a material that gradually decomposes, dissolves, hydrolyzes and/or erodes in situ. Generally, the "bioerodible" polymers herein are polymers that are hydrolyzable, and bioerode in situ primarily through hydrolysis.

The term "thixotropic" is used in its conventional sense to refer to a gel composition that can liquefy or at least exhibit a decrease in apparent viscosity upon application of mechanical force such as shear force. The extent of the reduction is in part a function of the shear rate of the gel when subjected to the shearing force. When the shearing force is removed, the viscosity of the thixotropic gel returns to a viscosity at or near that which it displayed prior to being subjected to the shearing force. Accordingly, a thixotropic gel may be subjected to a shearing force when injected from a syringe which temporarily reduces its viscosity during the injection process. When the injection process is completed, the shearing force is removed and the gel returns very near to its previous state.

A "thixotropic agent" as used herein is one that increases the thixotropy of the composition in which it is contained, promoting shear thinning and enabling use of reduced injection force.

The polymer, solvent and other agents of the invention must be "biocompatible"; that is they must not cause irritation, inflammation or necrosis in the environment of use. The environment of use is a fluid environment and may comprise a subcutaneous, intramuscular, intravascular (high/low flow), intramyocardial, adventitial, intratumoral, or intracerebral portion, wound sites, tight joint spaces or body cavity of a human or animal.

The following definitions apply to the molecular structures described herein:

As used herein, the phrase "having the formula" or "having the structure" is not intended to be limiting and is used in the same way that the term "comprising" is commonly used.

The term "alkyl" as used herein refers to a saturated hydrocarbon group typically although not necessarily containing 1 to about 30 carbon atoms, such as methyl, ethyl, n-propyl, isopropyl, n-butyl, isobutyl, t-butyl, octyl, decyl, and the like, as well as cycloalkyl groups such as cyclopentyl, cyclohexyl and the like. Generally, although again not necessarily, alkyl groups herein contain 1 to about 12 carbon atoms. The term "lower alkyl" intends an alkyl group of 1 to 6 carbon atoms, preferably 1 to 4 carbon atoms. "Substituted alkyl" refers to alkyl substituted with one or more

substituent groups, and the terms "heteroatom-containing alkyl" and "heteroalkyl" refer to alkyl in which at least one carbon atom is replaced with a heteroatom. If not otherwise indicated, the terms "alkyl" and "lower alkyl" include linear, branched, cyclic, unsubstituted, substituted, and/or heteroatom-containing alkyl or lower alkyl.

The term "aryl" as used herein, and unless otherwise specified, refers to an aromatic substituent containing a single aromatic ring or multiple aromatic rings that are fused together, linked covalently, or linked to a common group such as a methylene or ethylene moiety. Preferred aryl groups contain one aromatic ring or two fused or linked aromatic rings, e.g., phenyl, naphthyl, biphenyl, diphenylether, diphenylamine, benzophenone, and the like, and most preferred aryl groups are monocyclic. "Substituted aryl" refers to an aryl moiety substituted with one or more substituent groups, and the terms "heteroatom-containing aryl" and "heteroaryl" refer to aryl in which at least one carbon atom is replaced with a heteroatom. Unless otherwise indicated, the term "aryl" includes heteroaryl, substituted aryl, and substituted heteroaryl groups.

The term "aralkyl" refers to an alkyl group substituted with an aryl group, wherein alkyl and aryl are as defined above. The term "heteroaralkyl" refers to an alkyl group substituted with a heteroaryl group. Unless otherwise indicated, the term "aralkyl" includes heteroaralkyl and substituted aralkyl groups as well as unsubstituted aralkyl groups. Generally, the term "aralkyl" herein refers to an aryl-substituted lower alkyl group, preferably a phenyl substituted lower alkyl group such as benzyl, phenethyl, 1-phenylpropyl, 2-phenylpropyl, and the like.

The term "heteroatom-containing" as in a "heteroatom-containing hydrocarbyl group" refers to a molecule or molecular fragment in which one or more carbon atoms is replaced with an atom other than carbon, e.g., nitrogen, oxygen, sulfur, phosphorus or silicon. Similarly, the term "heterocyclic" refers to a cyclic substituent that is heteroatom-containing, the term "heteroaryl" refers to an aryl substituent that is heteroatom-containing, and the like.

By "substituted" as in "substituted alkyl," "substituted aryl" and the like, as alluded to in some of the aforementioned definitions, is meant that in the alkyl or aryl moiety, respectively, at least one hydrogen atom bound to a carbon atom is replaced with one or more non-interfering substituents such as hydroxyl, alkoxy, thio, amino, halo, and the like.

The Bioerodible, Biocompatible Polymer:

Polymers that are useful in conjunction with the methods and compositions of the invention are bioerodible, i.e., they gradually hydrolyze, dissolve, physically erode , or otherwise disintegrate within the aqueous fluids of a patient's body. Generally, the polymers bioerode as a result of hydrolysis or physical erosion, although the primary bioerosion process is typically hydrolysis.

Such polymers include, but are not limited to, polylactides, polyglycolides, polycaprolactones, polyanhydrides, polyamines, polyurethanes, polyesteramides, polyorthoesters, polydioxanones, polyacetals, polyketals, polycarbonates, polyphosphoesters, polyorthocarbonates, polyphosphazenes, succinates, poly(malic acid), poly(amino acids), polyvinylpyrrolidone, polyethylene glycol, polyhydroxycellulose, chitin, chitosan, hyaluronic acid, and copolymers, terpolymers and mixtures thereof.

Presently preferred polymers are polylactides, that is, a lactic acid-based polymer that can be based solely on lactic acid or can be a copolymer based on lactic acid glycolic acid and/or caprolactone, and which may include small amounts of other comonomers that do not substantially affect the advantageous results that can be achieved in accordance with the present invention. As used herein, the term "lactic acid" includes the isomers L-lactic acid, D-lactic acid, DL-lactic acid and lactide, while the term "glycolic acid" includes glycolide. Most preferred are poly(lactide-co-glycolide) copolymers, commonly referred to as "PLGA." The polymer may have a monomer ratio of lactic acid/glycolic acid of from about 100:0 to about 15:85, preferably from about 75:25 to about 30:70, more preferably from about 60:40 to about 40:60, and an especially useful copolymer has a monomer ratio of lactic acid/glycolic acid of about 50:50.

The poly(caprolactone-co-lactic acid) (PCL-co-LA) polymer has a comonomer ratio of caprolactone/lactic acid of from about 10:90 to about 90:10, from about 50:50; preferably from about 35:65 to about 65:35; and more preferably from about 25:75 to about 75:25. In certain embodiments, the lactic acid based polymer comprises a blend of about 0-90% caprolactone, about 0-100% lactic acid, and about 0-60% glycolic acid.

The lactic acid-based polymer has a number average molecular weight of from about 1,000 to about 120,000, preferably from about 5,000 to about 50,000, more preferably from about 8,000 to about 30,000, as determined by gel permeation chromatography (GPC). In contrast to prior polymer-based injectable depots, the present invention allows use of higher molecular weight polymers, insofar as the aromatic alcohol of the composition provides excellent shear thinning even with high molecular weight polymers. As indicated in aforementioned U.S. Patent No. 5,242,910, the polymer can be prepared in accordance with the teachings of U.S. Patent No. 4,443,340. Alternatively, the lactic acid-based polymer can be prepared directly from lactic acid or a mixture of lactic acid and glycolic acid (with or without a further comonomer) in accordance with the techniques set forth in U.S. Patent No. 5,310,865. The contents of all of these patents are incorporated by reference. Suitable lactic acid-based polymers are available commercially. For instance, 50:50 lactic acid:glycolic acid copolymers having molecular weights of 8,000, 10,000, 30,000 and 100,000 are available from Boehringer Ingelheim (Petersburg, VA), Medisorb Technologies International L.P. (Cincinatti, OH) and

Birmingham Polymers, Inc. (Birmingham, AL) as described below.

Examples of polymers include, but are not limited to, Poly (D,L-lactide) Resomer® L104, PLA-L104, Poly (D, L-lactide-co-glycolide) 50:50 Resomer® RG502, Poly (D,L-lactide-co-glycolide) 50:50 Resomer® RG502H, PLGA-502H, Poly (D,L-lactide-co-glycolide) 50:50 Resomer® RG503, PLGA-503, Poly (D,L-lactide-co-glycolide) 50:50 Resomer® RG506, PLGA-506, Poly L-Lactide MW 2,000 (Resomer® L 206, Resomer® L 207, Resomer® L 209, Resomer® L 214); Poly D,L Lactide (Resomer® R 104, Resomer® R 202, Resomer® R 203, Resomer® R 206, Resomer® R 207, Resomer® R 208); Poly L-Lactide-co-D,L-lactide 90:10 (Resomer® LR 209); Poly glycolide (Resomer® G 205); Poly D,L-lactide-co-glycolide 50:50 (Resomer® RG 504 H, Resomer® RG 504, Resomer® RG 505); Poly D-L-lactide-co-glycolide 75:25 (Resomer® RG 752, , PLGA-755, Resomer® RG 756); Poly D,L-lactide-co-glycolide 85:15 (Resomer® RG 858); Poly L-lactide-co-trimethylene carbonate 70:30 (Resomer® LT 706); Poly dioxanone (Resomer® X 210) (Boehringer Ingelheim Chemicals, Inc., Petersburg, VA).

Additional examples include, but are not limited to, DL-lactide/glycolide 100:0 (MEDISORB® Polymer 100 DL High, MEDISORB® Polymer 100 DL Low); DL-lactide/ glycolide 85/15 (MEDISORB® Polymer 8515 DL High, MEDISORB® Polymer 8515 DL Low); DL-lactide/glycolide 75/25 (MEDISORB® Polymer 7525 DL High, MEDISORB® Polymer 7525 DL Low); DL-lactide/glycolide 65/35 (MEDISORB® Polymer 6535 DL High, MEDISORB® Polymer 6535 DL Low); DL-lactide/glycolide 54/46 (MEDISORB® Polymer 5050 DL High, MEDISORB® Polymer 5050 DL Low); and DL-lactide/glycolide 54/46 (MEDISORB® Polymer 5050 DL 2A(3), MEDISORB® Polymer 5050 DL 3A(3), MEDISORB® Polymer 5050 DL 4A(3)) (Medisorb Technologies International L.P., Cincinatti, OH); and Poly D,L-lactide-co-glycolide 50:50; Poly D,L-lactide-co-glycolide 65:35; Poly D,L-lactide-co-glycolide 75:25; Poly D,L-lactide-co-glycolide 85:15; Poly DL-lactide; Poly L-lactide; Poly glycolide; Poly ε-caprolactone; Poly DL-lactide-co-caprolactone 25:75; and Poly DL-lactide-co-caprolactone 75:25 (Birmingham Polymers, Inc., Birmingham, AL).

The biocompatible polymer is present in the gel composition in an amount ranging from 35 to 75%, by weight of the viscous gel, the viscous gel comprising the combined amounts of the biocompatible polymer and the aromatic alcohol. The solvent will be added to polymer in amounts described below, to provide implantable or viscous gels. Again, the aromatic alcohol enables a much wider range of polymer/solvent ratios than obtainable previously.

Solvents and Thixotropic Agents:

In a first embodiment, the injectable depot composition of the invention contains a water-immiscible aromatic alcohol in addition to the bioerodible polymer and the beneficial agent. In this embodiment, the aromatic alcohol serves as a solvent and as a thixotropic agent, facilitating solubilization of the bioerodible polymer and also promoting shear thinning behavior upon injection. The composition is free of monohydric lower alkanols, as such solvents are volatile, causing problems during manufacture, and are potentially denaturing to or otherwise reactive with the beneficial agent. Preferably the compositions described herein are also free of solvents having miscibility in water that is greater than 7 wt.% at 25°C.

The aromatic alcohol must be biocompatible, should form a viscous gel with the polymer, and restrict water uptake into the implant. Suitable aromatic alcohols will substantially restrict the uptake of water by the implant and, as noted above, may be characterized as immiscible in water, i.e., having a solubility or miscibility in water of at most 7% by weight. Preferably, the water solubility of the aromatic alcohol is 5 wt.% or less, more preferably 3 wt.% or less, and even more preferably 1 wt.% or less. Most preferably, the solubility of the aromatic alcohol in water is equal to or less than 0.5 weight percent.

Water miscibility may be determined experimentally as follows: Water (1-5 g) is placed in a tared clear container at a controlled temperature, about 25°C, and weighed, and a candidate solvent is added dropwise. The solution is swirled to observe phase separation. When the saturation point appears to be reached, as determined by observation of phase separation, the solution is allowed to stand overnight and is re-checked the following day. If the solution is still saturated, as determined by observation of phase separation, then the percent (w/w) of solvent added is determined. Otherwise more solvent is added and the process repeated. Solubility or miscibility is determined by dividing the total weight of solvent added by the final weight of the solvent/water mixture. When solvent mixtures are used, they are premixed prior to adding to the water.

The aromatic alcohol preferably as the structural formula (I)

$$Ar\text{-}(L)_n\text{-}OH \text{ (I)} \qquad \text{(I)}$$

wherein Ar is a substituted or unsubstituted aryl or heteroaryl group, n is zero or 1, and L is a linking moiety. Preferably, Ar is a monocyclic aryl or heteroaryl group, optionally substituted with one or more noninterfering substituents such as hydroxyl, alkoxy, thio, amino, halo, and the like. More preferably, Ar is an unsubstituted 5- or 6-membered aryl or heteroaryl group such as phenyl, cyclopentadienyl, pyridinyl, pyrimadinyl, pyrazinyl, pyrrolyl, pyrazolyl, imidazolyl, furanyl, thiophenyl, thiazolyl, isothiazolyl, or the like. The subscript "n" is zero or 1, meaning that the linking moiety L may or may

not be present. Preferably, n is 1 and L is generally a lower alkylene linkage such as methylene or ethylene, wherein the linkage may include heteroatoms such as O, N or S. Most preferably, Ar is phenyl, n is 1, and L is methylene, such that the aromatic alcohol is benzyl alcohol.

In another embodiment, the injectable depot composition of the invention contains, in addition to the biocompatible, bioerodible polymer and the beneficial agent, (1) a solvent selected from the group consisting of esters of aromatic acids, aromatic ketones, and mixtures thereof, which has miscibility in water of less than or equal to 7% at 25°C, and is present in an amount effective to plasticize the polymer and form a gel therewith, and (2) an effective thixotropic amount of an aromatic alcohol as described above. Generally, the weight ratio of the aromatic alcohol to the ester or ketone is in the range of about 1% to about 99%, preferably in the range of about 10% to about 90%, preferably in the range of about 20% to about 80%, preferably in the range of about 25% to about 75%, often in the range of about 25% to about 50%. In this case, the aromatic alcohol serves primarily as a thixotropic agent, but also acts as a co-solvent for the bioerodible polymer. Like the injectable composition of the first embodiment, this composition is also free of monohydric lower alkanols.

The aromatic acid ester or ketone must be biocompatible, should form a viscous gel with the polymer, and restrict water uptake into the implant. Like the aromatic alcohol, suitable aromatic acid esters and ketones will substantially restrict the uptake of water by the implant and, as noted above, may be characterized as immiscible in water, i.e., having a solubility or miscibility in water of at most 7% by weight. Preferably, the water solubility of the solvent alcohol is 5 wt. % or less, more preferably 3 wt.% or less, and even more preferably 1 wt.% or less. Most preferably, the solubility of the solvent in water is equal to or less than 0.5 weight percent.

The aromatic acid ester or ketone may be selected from the lower alkyl and aralkyl esters of aromatic acids, and aryl and aralkyl ketones. Generally, although not necessarily, the aromatic acid esters and ketones will respectively have the structural formula (II) or (III)

(II) 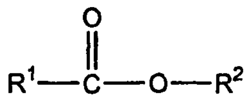

(III) 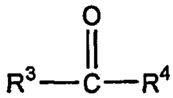

In the ester of formula (II), $R^1$ is substituted or unsubstituted aryl, aralkyl, heteroaryl or heteroaralkyl, preferably substituted or unsubstituted aryl or heteroaryl, more preferably monocyclic or bicyclic aryl or heteroaryl optionally substituted with one or more non-interfering substituents such as hydroxyl, carboxyl, alkoxy, thio, amino, halo, and the like, still more preferably 5- or 6-membered aryl or heteroaryl such as phenyl, cyclopentadienyl, pyridinyl, pyrimadinyl, pyrazinyl, pyrrolyl, pyrazolyl, imidazolyl, furanyl, thiophenyl, thiazolyl, or isothiazolyl, and most preferably 5- or 6-membered aryl. $R^2$ is hydrocarbyl or heteroatom-substituted hydrocarbyl, typically lower alkyl or substituted or unsubstituted aryl, aralkyl, heteroaryl or heteroaralkyl, preferably lower alkyl or substituted or unsubstituted aralkyl or heteroaralkyl, more preferably lower alkyl or monocyclic or bicyclic aralkyl or heteroaralkyl optionally substituted with one or more non-interfering substituents such as hydroxyl, carboxyl, alkoxy, thio, amino, halo, and the like, still more preferably lower alkyl or 5- or 6-membered aralkyl or heteroaralkyl, and most preferably lower alkyl or 5- or 6-membered aryl optionally substituted with one or more additional ester groups having the structure -O-(CO)-$R^1$. Most preferred esters are benzoic acid and phthalic acid derivatives.

In the ketone of formula (III), $R^3$ and $R^4$ may be selected from any of the $R^1$ and $R^2$ groups identified above.

Art recognized benzoic acid derivatives from which solvents having the requisite solubility may be selected include, without limitation: 1,4-cyclohexane dimethanol dibenzoate, diethylene glycol dibenzoate, dipropylene glycol dibenzoate, polypropylene glycol dibenzoate, propylene glycol dibenzoate, diethylene glycol benzoate and dipropylene glycol benzoate blend, polyethylene glycol (200) dibenzoate, isodecyl benzoate, neopentyl glycol dibenzoate, glyceryl tribenzoate, pentaerylthritol tetrabenzoate, cumylphenyl benzoate, trimethyl pentanediol dibenzoate.

Art recognized phthalic acid derivatives from which solvents having the requisite solubility may be selected include: Alkyl benzyl phthalate, bis-cumyl-phenyl isophthalate, dibutoxyethyl phthalate, dimethyl phthalate, dimethyl phthalate, diethyl phthalate, dibutyl phthalate, diisobutyl phthalate, butyl octyl phthalate, diisoheptyl phthalate, butyl octyl

phthalate, diisononyl phthalate, nonyl undecyl phthalate, dioctyl phthalate, di-isooctyl phthalate, dicapryl phthalate, mixed alcohol phthalate, di-(2-ethylhexyl) phthalate, linear heptyl, nonyl, phthalate, linear heptyl, nonyl, undecyl phthalate, linear nonyl phthalate, linear nonyl undecyl phthalate, linear dinonyl, didecyl phthalate (diisodecyl phthalate), diundecyl phthalate, ditridecyl phthalate, undecyldodecyl phthalate, decyltridecyl phthalate, blend (50/50) of dioctyl and didecyl phthalates, butyl benzyl phthalate, and dicyclohexyl phthalate.

Most preferred solvents are derivatives of benzoic acid and include, but are not limited to, methyl benzoate, ethyl benzoate, n-propyl benzoate, isopropyl benzoate, butyl benzoate, isobutyl benzoate, sec-butyl benzoate, tert-butyl benzoate, isoamyl benzoate and benzyl benzoate, with benzyl benzoate being most especially preferred.

The composition may also include, in addition to the water-immiscible solvent(s), one or more additional miscible solvents ("component solvents"), provided that any such additional solvent is other than a lower alkanol. Component solvents compatible and miscible with the primary solvent(s) may have a higher miscibility with water and the resulting mixtures may still exhibit significant restriction of water uptake into the implant. Such mixtures will be referred to as "component solvent mixtures." Useful component solvent mixtures may exhibit solubilities in water greater than the primary solvents themselves, typically between 0.1 weight percent and up to and including 50 weight percent, preferably up to and including 30 weight percent, and most preferably up to an including 10 weight percent, without detrimentally affecting the restriction of water uptake exhibited by the implants of the invention.

Component solvents useful in component solvent mixtures are those solvents that are miscible with the primary solvent or solvent mixture, and include, but are not limited, to triacetin, diacetin, tributyrin, triethyl citrate, tributyl citrate, acetyl triethyl citrate, acetyl tributyl citrate, triethylglycerides, triethyl phosphate, diethyl phthalate, diethyl tartrate, mineral oil, polybutene, silicone fluid, glycerin, ethylene glycol, polyethylene glycol, octanol, ethyl lactate, propylene glycol, propylene carbonate, ethylene carbonate, butyrolactone, ethylene oxide, propylene oxide, N-methyl-2-pyrrolidone, 2-pyrrolidone, glycerol formal, methyl acetate, ethyl acetate, methyl ethyl ketone, dimethylformamide, glycofurol, dimethyl sulfoxide, tetrahydrofuran, caprolactam, decylmethylsulfoxide, oleic acid, and 1-dodecylazacyclo-heptan-2-one, and mixtures thereof.

In an especially preferred embodiment, the solvent is selected from lower alkyl and aralkyl esters of benzoic acid, the aromatic alcohol is present, serving as a thixotropic agent, and the polymer is a lactic-acid based polymer, most preferably PLGA, having a number average molecular weight of between about 1,000 to about 120,000, preferably about 5,000 to 50,000, more preferably about 8,000 to 30,000. Presently, the most preferred solvents are benzyl benzoate and the lower alkyl esters of benzoic acid, the most preferred thixotropic agent is benzyl alcohol, as noted earlier herein.

The solvent or solvent mixture is capable of dissolving the polymer to form a viscous gel that can maintain particles of the beneficial agent dissolved or dispersed and isolated from the environment of use prior to release. The compositions of the present invention provide implants having a low burst index. Water uptake is controlled by the use of a solvent or component solvent mixture that solublizes or plasticizes the polymer but substantially restricts uptake of water into implant.

The solvent or solvent mixture is typically present in an amount of from about 95 to about 5% by weight, preferably about 75 to about 15% by weight, and most preferably about 65% to about 20% by weight of the viscous gel. The viscous gel formed by mixing the polymer and the solvent typically exhibits a viscosity of from about 10 to about 200,00 Pa·s, preferably from about 50 to about 5000 Pa·s, often from about 100 to about 5000 Pa·s measured at a 1 $sec^{-1}$ shear rate and 25°C using a Haake Rheometer at about 1-2 days after mixing is completed. Mixing the polymer with the solvent can be achieved with conventional low shear equipment such as a Ross double planetary mixer for from about 10 minutes to about 1 hour, although shorter and longer periods may be chosen by one skilled in the art depending on the particular physical characteristics of the composition being prepared. Since it is often desirable to administer the implant as an injectable composition, a countervailing consideration when forming implants that are viscous gels is that the polymer/solvent/beneficial agent composition have sufficiently low viscosity in order to permit it to be forced through a small diameter, e.g., 16 gauge and higher, preferably 20 gauge and higher, more preferably 22 gauge and higher, even more preferably 24 gauge and higher gauge needle. If necessary, adjustment of viscosity of the gel for injection can be accomplished with emulsifying agents as described herein. Yet, such compositions should have adequate dimensional stability so as to remain localized and be able to be removed if necessary. The particular gel or gel-like compositions of the present invention satisfy such requirements.

Beneficial Agents:

The beneficial agent can be any physiologically or pharmacologically active substance or substances optionally in combination with pharmaceutically acceptable carriers and additional ingredients such as antioxidants, stabilizing agents, permeation enhancers, etc. that do not substantially adversely affect the advantageous results that can be attained by the present invention. The beneficial agent may be any of the agents which are known to be delivered to the body of a human or an animal and that are preferentially soluble in water rather than in the polymer-dissolving solvent. These agents include drug agents, medicaments, vitamins, nutrients, or the like. Included among the types of agents

which meet this description are lower molecular weight compounds, proteins, peptides, genetic material, nutrients, vitamins, food supplements, sex sterilants, fertility inhibitors and fertility promoters.

Drug agents which may be delivered by the present invention include drugs which act on the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autacoid systems, the alimentary and excretory systems, the histamine system and the central nervous system. Suitable agents may be selected from, for example, a drug, proteins, enzymes, hormones, polynucleotides, nucleoproteins, polysaccharides, glycoproteins, lipoproteins, polypeptides, steroids, analgesics, local anesthetics, antibiotic agents, chemotherapeutic agents, immunosuppressive agents, anti-inflammatory agents including anti-inflammatory corticosteroids, antiproliferative agents, antimitotic agents, angiogenic agents, anticoagulants, fibrinolytic agents, growth factors, antibodies, ocular drugs, and metabolites, analogs (including synthetic and substituted analogs), derivatives (including aggregative conjugates/fusion with other macromolecules and covalent conjugates with unrelated chemical moieties by means known in the art) fragments, and purified, isolated, recombinant and chemically synthesized versions of these species.

Examples of drugs that may be delivered by the composition of the present invention include, but are not limited to, procaine, procaine hydrochloride, tetracaine, tetracaine hydrochloride, cocaine, cocaine hydrochloride, chloroprocaine, chloroprocaine hydrochloride, proparacaine, proparacaine hydrochloride, piperocaine, piperocaine hydrochloride, hexylcaine, hexylcaine hydrochloride, naepaine, naepaine hydrochloride, benzoxinate, benzoxinate hydrochloride, cyclomethylcaine, cyclomethylcaine hydrochloride, cyclomethylcaine sulfate, lidocaine, lidocaine hydrochloride, bupivicaine, bupivicaine hydrochloride, mepivicaine, mepivacaine hydrochloride, prilocaine, prilocaine hydrochloride, dibucaine and dibucaine hydrochloride, etidocaine, benzocaine, propoxycaine, dyclonin, pramoxine, oxybuprocaine, prochlorperzine edisylate, ferrous sulfate, aminocaproic acid, mecamylamine hydrochloride, procainamide hydrochloride, amphetamine sulfate, methamphetamine hydrochloride, benzamphetamine hydrochloride, isoproterenol sulfate, phenmetrazine hydrochloride, bethanechol chloride, methacholine chloride, pilocarpine hydrochloride, atropine sulfate, scopolamine bromide, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperzine maleate, anisindone, diphenadione erythrityl tetranitrate, digoxin, isoflurophate, acetazolamide, methazolamide, bendroflumethiazide, chloropromaide, tolazamide, chlormadinone acetate, phenaglycodol, allopurinol, aluminum aspirin, methotrexate, acetyl sulfisoxazole, erythromycin, hydrocortisone, hydrocorticosterone acetate, cortisone acetate, dexamethasone and its derivatives such as betamethasone, triamcinolone, methyltestosterone, 17-S-estradiol, ethinyl estradiol, ethinyl estradiol 3-methyl ether, prednisolone, 17$\alpha$-hydroxyprogesterone acetate, 19-nor-progesterone, norgestrel, norethindrone, norethisterone, norethiederone, progesterone, norgesterone, norethynodrel, aspirin, indomethacin, naproxen, fenoprofen, sulindac, indoprofen, nitroglycerin, isosorbide dinitrate, propranolol, timolol, atenolol, alprenolol, cimetidine, clonidine, imipramine, levodopa, chlorpromazine, methyldopa, dihydroxyphenylalanine, theophylline, calcium gluconate, ketoprofen, ibuprofen, cephalexin, erythromycin, haloperidol, zomepirac, ferrous lactate, vincamine, diazepam, phenoxybenzamine, diltiazem, milrinone, mandol, quanbenz, hydrochlorothiazide, ranitidine, flurbiprofen, fenufen, fluprofen, tolmetin, alclofenac, mefenamic, flufenamic, difuinal, nimodipine, nitrendipine, nisoldipine, nicardipine, felodipine, lidoflazine, tiapamil, gallopamil, amlodipine, mioflazine, lisinolpril, enalapril, enalaprilat, captopril, ramipril, famotidine, nizatidine, sucralfate, etintidine, tetratolol, minoxidil, chlordiazepoxide, diazepam, amitriptyline, and imipramine. Further examples are proteins and peptides which include, but are not limited to, bone morphogenic proteins, insulin, colchicine, glucagon, thyroid stimulating hormone, parathyroid and pituitary hormones, calcitonin, renin, prolactin, corticotrophin, thyrotropic hormone, follicle stimulating hormone, chorionic gonadotropin, gonadotropin releasing hormone, bovine somatotropin, porcine somatotropin, oxytocin, vasopressin, GRF, somatostatin, lypressin, pancreozymin, luteinizing hormone, LHRH, LHRH agonists and antagonists, leuprolide, interferons such as interferon alpha-2a, interferon alpha-2b, and consensus interferon, interleukins, growth factors such as epidermal growth factors (EGF), platelet-derived growth factors (PDGF), fibroblast growth factors (FGF), transforming growth factors-$\alpha$ (TGF-$\alpha$), transforming growth factors-$\beta$ (TGF-$\beta$), erythropoietin (EPO), insulin-like growth factor-I (IGF-I), insulin-like growth factor-II (IGF-II), interleukin-1, interleukin-2, interleukin-6, interleukin-8, tumor necrosis factor-$\alpha$ (TNF-$\alpha$), tumor necrosis factor-$\beta$ (TNF-$\beta$), Interferon-$\alpha$ (INF-$\alpha$), Interferon-$\beta$ (INF-$\beta$), interferon-$\gamma$ (INF-$\gamma$), Interteron-$\omega$ (INF-$\omega$), colony stimulating factors (CGF), vascular cell growth factor (VEGF), thrombopoietin (TPO), stromal cell-derived factors (SDF), placenta growth factor (PIGF), hepatocyte growth factor (HGF), granulocyte macrophage colony stimulating factor (GM-CSF), glial-derived neurotropin factor (GDNF), granulocyte colony stimulating factor (G-CSF), ciliary neurotropic factor (CNTF), bone morphogeneic proteins (BMP), coagulation factors, human pancreas hormone releasing factor, analogs and derivatives of these compounds, and pharmaceutically acceptable salts of these compounds, or their analogs or derivatives.

Additional examples of drugs that may be delivered by the composition of the present invention include, but are not limited to, antiproliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin, actinomycin D, daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP)$II_bIII_a$ inhibitors and vitronectin receptor antagonists; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes - dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine (cladribine)); platinum coordination complexes (cisplatin, carboplatin), procarbazine, hydroxyurea, mitotane, aminoglutethimide; hormones (i.e. estrogen); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); antiinflammatory: such as adrenocortical steroids (cortisol, cortisone, fludrocortisone, prednisone, prednisolone, 6α-methylprednisolone, triamcinolone, betamethasone, and dexamethasone), non-steroidal agents (salicylic acid derivatives i.e. aspirin; para-aminophenol derivatives i.e. acetominophen); indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF); angiotensin receptor blocker; nitric oxide donors; anti-sense oligionucleotides and combinations thereof; cell cycle inhibitors, mTOR inhibitors, and growth factor signal transduction kinase inhibitors, analogs and derivatives of these compounds, and pharmaceutically acceptable salts of these compounds, or their analogs or derivatives.

In certain preferred embodiments, the beneficial agent includes chemotactic growth factors, proliferative growth factors, stimulatory growth factors, and transformational peptide growth factors including genes, precursors, post-translational-variants, metabolites, binding-proteins, receptors, receptor agonists and antagonists of the following growth factor families: epidermal growth factors (EGFs), platelet-derived growth factor (PDGFs), insulin-like growth factors (IGFs), fibroblast-growth factors (FGFs), transforming-growth factors (TGFs), interleukins (ILs), colony-stimulating factors (CSFs, MCFs, GCSFs, GMCSFs), Interferons (IFNs), endothelial growth factors (VEGF, EGFs), erythropoietins (EPOs), angiopoietins (ANGs), placenta-derived growth factors (PIGFs), and hypoxia induced transcriptional regulators (HIFs).

The present invention also finds application with chemotherapeutic agents for the local application of such agents to avoid or minimize systemic side effects. Gels of the present invention containing chemotherapeutic agents may be injected directly into the tumor tissue for sustained delivery of the chemotherapeutic agent over time. In some cases, particularly after resection of the tumor, the gel may be implanted directly into the resulting cavity or may be applied to the remaining tissue as a coating. In cases in which the gel is implanted after surgery, it is possible to utilize gels having higher viscosities since they do not have to pass through a small diameter needle. Representative chemotherapeutic agents that may be delivered in accordance with the practice of the present invention include, for example, carboplatin, cisplatin, paclitaxel, BCNU, vincristine, camptothecin, etopside, cytokines, ribozymes, interferons, oligonucleotides and oligonucleotide sequences that inhibit translation or transcription of tumor genes, functional derivatives of the foregoing, and generally known chemotherapeutic agents such as those described in U.S. Patent 5,651,986. The present application has particular utility in the sustained delivery of water soluble chemotherapeutic agents, such as for example cisplatin and carboplatin and the water soluble derivatives of paclitaxel. Those characteristics of the invention that minimize the burst effect are particularly advantageous in the administration of water soluble beneficial agents of all kinds, but particularly those compounds that are clinically useful and effective but may have adverse side effects.

To the extent not mentioned above, the beneficial agents described in aforementioned U.S. Patent No. 5,242,910 can also be used. One particular advantage of the present invention is that materials, such as proteins, as exemplified by the enzyme lysozyme, and cDNA, and DNA incorporated into vectors both viral and nonviral, which are difficult to microencapsulate or process into microspheres can be incorporated into the compositions of the present invention without the level of degradation caused by exposure to high temperatures and denaturing solvents often present in other processing techniques.

The beneficial agent is incorporated into the viscous gel formed from the polymer and the solvent in the form of particles typically having an average particle size of from about 0.1 to about 250 microns, preferably from about 1 to about 200 microns and often from 30 to 125 microns. For instance, particles having an average particle size of about 5 microns have been produced by spray drying or freeze drying an aqueous mixture containing 50% sucrose and 50% chicken lysozyme (on a dry weight basis) and mixtures of 10-20% hGH and 15-30 mM zinc acetate. Such particles have been used in certain of the examples illustrated in the figures. Conventional lyophilization processes can also be utilized to form particles of beneficial agents of varying sizes using appropriate freezing and drying cycles.

To form a suspension or dispersion of particles of the beneficial agent in the viscous gel formed from the polymer and the solvent, any conventional low shear device can be used such as a Ross double planetary mixer at

ambient conditions. In this manner, efficient distribution of the beneficial agent can be achieved substantially without degrading the beneficial agent.

The beneficial agent is typically dispersed in the composition in an amount of from about 0.1% to about 50% by weight, preferably in an amount of from about 1% to about 40%, more preferably in an amount of about 2% to about 30%, and often 2 to 20% by weight of the combined amounts of the polymer, solvent, and beneficial agent. Depending on the amount of beneficial agent present in the composition, one can obtain different release profiles and burst indices. More specifically, for a given polymer and solvent, by adjusting the amounts of these components and the amount of the beneficial agent, one can obtain a release profile that depends more on the degradation of the polymer than the diffusion of the beneficial agent from the composition or vice versa. In this respect, at lower beneficial agent loading rates, one generally obtains a release profile reflecting degradation of the polymer wherein the release rate increases with time. At higher loading rates, one generally obtains a release profile caused by diffusion of the beneficial agent wherein the release rate decreases with time. At intermediate loading rates, one obtains combined release profiles so that if desired, a substantially constant release rate can be attained. In order to minimize burst, loading of beneficial agent on the order of 30% or less by weight of the overall gel composition, i.e., polymer, solvent and beneficial agent, is preferred, and loading of 20% or less is more preferred.

Release rates and loading of beneficial agent will be adjusted to provide for therapeutically effective delivery of the beneficial agent over the intended sustained delivery period. Preferably, the beneficial agent will be present in the polymer gel at concentrations that are above the saturation concentration of beneficial agent in water to provide a drug reservoir from which the beneficial agent is dispensed. While the release rate of beneficial agent depends on the particular circumstances, such as the beneficial agent to be administered, release rates on the order of from about 0.1 micrograms/day to about 30 milligrams/day, preferably from about 1 microgram/day to about 20 milligrams per day, more preferably from about 10 micrograms/day to about 10 milligram/day, for periods of from about 24 hours to about 180 days, preferably 24 hours to about 120 days, more preferably 24 hours to about 90 days, often 3 days to about 90 days can be obtained. Further, the dose of beneficial agent may be adjusted by adjusting the amount of depot gel injected. Greater amounts may be delivered if delivery is to occur over shorter periods. Generally, higher release rate is possible if a greater burst can be tolerated. In instances where the gel composition is surgically implanted, or used as a "leave behind" depot when surgery to treat the disease state or another condition is concurrently conducted, it is possible to provide higher doses that would normally be administered if the implant was injected. Further, the dose of beneficial agent may be controlled by adjusting the volume of the gel implanted or the injectable gel injected. Preferably, the system releases 40% or less by weight of the beneficial agent present in the viscous gel within the first 24 hours after implantation in the subject. More preferably, 30% or less by weight of the beneficial agent will be released within the first 24 hours after implantation, and the implanted composition has a burst index of 12 or less, preferably 8 or less.

Optional Additional Components:

Other components may be present in the gel composition, to the extent they are desired or provide useful properties to the composition, such as polyethylene glycol, hydroscopic agents, stabilizing agents (for example surfactants like tween 20, tween 80, and the like, sugars such as sucrose, treholose, and the like, salts, antioxidants), pore forming agents, bulking agents (such as sorbitol, mannitol, glycine, and the like), chelating agents (such as divalent metal ions including zinc, magnesium, calcium, copper and the like), buffering agents (such as phosphate, acetane, succinate, histidine, TRIS, and the like) and others. When the composition includes a peptide or a protein that is soluble in or unstable in an aqueous environment, it may be highly desirable to include a solubility modulator that may, for example, be a stabilizing agent, in the composition. Various modulating agents are described in U.S. Patent Nos. 5,654,010 and 5,656,297. . In the case of hGH, for example, it is preferable to include an amount of a salt of a divalent metal, preferably zinc. Examples of such modulators and stabilizing agents, which may form complexes with the beneficial agent or associate to provide the stabilizing or modulated release effect, include metal cations, preferably divalent, present in the composition as magnesium carbonate, zinc carbonate, calcium carbonate, magnesium acetate, magnesium sulfate, zinc acetate, zinc sulfate, zinc chloride, magnesium chloride, magnesium oxide, magnesium hydroxide, other antacids, and the like. The amounts of such agents used will depend on the nature of the complex formed, if any, or the nature of the association between the beneficial agent and the agent. Molar ratios of solubility modulator or stabilizing agent to beneficial agent of about 100:1 to 1:1, preferably 10:1 1 to 1:1, typically can be utilized.

Pore forming agents include biocompatible materials that when contacted with body fluids dissolve, disperse or degrade to create pores or channels in the polymer matrix. Typically, organic and non-organic materials that are water soluble such as sugars (e.g., sucrose, dextrose), water soluble salts (e.g., sodium chloride, sodium phosphate, potassium chloride, and sodium carbonate), water soluble solvents such as N-methyl-2-pyrrolidone and polyethylene glycol and water soluble polymers (e.g., carboxymethylcellulose, hydroxypropyl-cellulose, and the like) can conveniently be used as pore formers. Such materials may be present in amounts varying from about 0.1 % to about 100% of the weight of the polymer, but will typically be less than 50% and more typically less than 10-20% of the weight of polymer.

Utility and Administration:

The means of administration of the implants is not limited to injection, although that mode of delivery may often be preferred. Where the implant will be administered as a leave-behind product, it may be formed to fit into a body cavity existing after completion of surgery or it may be applied as a flowable gel by brushing or palleting the gel onto residual tissue or bone. Such applications may permit loading of beneficial agent in the gel above concentrations typically present with injectable compositions.

To further understand the various aspects of the present invention, the results set forth in the previously described figures were obtained in accordance with the following examples.

EXAMPLE 1

A gel vehicle for use in an injectable depot of the composition was prepared as follows. A glass vessel was tared on a Mettler PJ3000 top loader balance. Poly (D,L-lactide-co-glycolide) (PLGA), available as 50:50 Resomer® RG502 (PLGA RG 502), was weighed into the glass vessel. The glass vessel containing PLGA was tared and the corresponding solvent was added. Amounts expressed as percentages for various polymer/solvent combinations are set forth in Table 1, below. The polymer/solvent mixture was manually stirred with a stainless steel square-tip spatula, resulting in a sticky amber paste-like substance containing white polymer particles. The vessel containing the polymer/ solvent mixture was sealed and placed in a temperature controlled incubator equilibrated to 39°C. The polymer/solvent mixture was removed from the incubator when it appeared to be a clear amber homogeneous gel. Incubation time intervals ranged from 1 to 4 days, depending on solvent and polymer type and solvent and polymer ratios. Thereafter, the mixture was placed in an oven (65°C) for 30 minutes. It was noted that the PLGA-504 was dissolved in the mixture upon removal from the oven.

Additional depot gel vehicles are prepared with the following solvents or mixtures: benzyl benzoate ("BB"), benzyl alcohol ("BA"), and propylene glycol ("PG"), and the following polymers: Poly (D,L-lactide) Resomer® L104, PLA-L104, Poly (D,L-lactide-co-glycolide) 50:50 Resomer® RG502, Poly (D,L-lactide-co-glycolide) 50:50 Resomer® RG502H, PLGA-502H, Poly (D,L-lactide-co-glycolide) 50:50 Resomer® RG503, PLGA-503, Poly L-Lactide MW 2,000 (Resomer® L 206, Resomer® L 207, Resomer® L 209, Resomer® L 214); Poly D,L Lactide (Resomer® R 104, Resomer® R 202, Resomer® R 203, Resomer® R 206, Resomer® R 207, Resomer® R 208); Poly L-Lactide-co-D,L-lactide 90:10 (Resomer® LR 209); Poly D-L-lactide-co-glycolide 75:25 (Resomer® RG 752, Resomer® RG755, Resomer® RG 756); Poly D,L-lactide-co-glycolide 85:15 (Resomer® RG 858); Poly L-lactide-co-trimethylene carbonate 70:30 (Resomer® LT 706); Poly dioxanone (Resomer® X 210) (Boehringer Ingelheim Chemicals, Inc., Petersburg, VA); DL-lactide/glycolide 100:0 (MEDISORB® Polymer 100 DL High, MEDISORB® Polymer 100 DL Low); DL-lactide/ glycolide 85/15 (MEDISORB® Polymer 8515 DL High, MEDISORB® Polymer 8515 DL Low); DL-lactide/glycolide 75/25 (MEDISORB® Polymer 7525 DL High, MEDISORB® Polymer 7525 DL Low); DL-lactide/glycolide 65/35 (MEDISORB® Polymer 6535 DL High, MEDISORB® Polymer 6535 DL Low); DL-lactide/glycolide 54/46 (MEDISORB® Polymer 5050 DL High, MEDISORB® Polymer 5050 DL Low); and DL-lactide/glycolide 54/46 (MEDISORB® Polymer 5050 DL 2A(3), MEDISORB® Polymer 5050 DL 3A(3), MEDISORB® Polymer 5050 DL 4A(3)) (Medisorb Technologies International L.P., Cincinatti, OH); and Poly D,L-lactide-co-glycolide 50:50; Poly D,L-lactide-co-glycolide 65:35; Poly D,L-lactide-co-glycolide 75:25; Poly D,L-lactide-co-glycolide 85:15; Poly DL-lactide; Poly L-lactide; Poly glycolide; Poly ε-caprolactone; Poly DL-lactide-co-caprolactone 25:75; and Poly DL-lactide-co-caprolactone 75:25 (Birmingham Polymers, Inc., Birmingham, AL). Representative gel vehicles are described in Table 1 below.

Table 1

| Formulation | Polymer gm (%) | Benzyl Benzoate gm (%) | Benzyl Alcohol gm (%) | PG gm (%) |
|---|---|---|---|---|
| 1 | 5.0365 | 4.5093 | 0.5178 | - |
| 2 | 5.0139 | 3.7553 | 1.2560 | - |
| 3 | 5.0350 | 4.5193 | - | 0.5206 |
| 4 | 5.0024 | 3.7547 | - | 1.2508 |
| 5 | 5.0068 | 5.0044 | - | - |

EXAMPLE 2

Rheological behavior was tested for depot vehicles formulated with different solvents. A vehicle comprising 50

wt.% polymer (PLGA RG502) and 50 wt.% solvent (benzyl alcohol) was prepared according to the procedures outlined in Example 1. For comparative purposes, solvent comprising benzyl benzoate (e.g., formulation 5) or benzyl benzoate combined with ethanol (e.g., formulation 7) were also prepared. Table 2 lists the formulations used in the test.

Table 2

| Formulation | Polymer (%) | Benzyl Benzoate (%) | Benzyl Alcohol (%) | Ethanol (%) |
|---|---|---|---|---|
| 5 Comparative | 50.0 | 50.0 | 0.0 | 0.0 |
| 6 | 50.0 | 0.0 | 50.0 | 0.0 |
| 7 Comparative | 45.0 | 52.8 | 0.0 | 2.2 |

Formulations 5, 6 and 7 were tested for viscosity under various shear rates. As indicated in Figure 1, significant shear thinning behavior was observed when benzyl alcohol was used as the solvent (e.g., formulation 6), in contrast to formulations using benzyl benzoate (e.g., formulation 5) and benzyl benzoate with ethanol (e.g., formulation 7) as a thixotropic agent, respectively.

EXAMPLE 3

The injection force required to dispense depot vehicles was evaluated for the three formulations identified in Example 2. The formulations were injected through a 24-gauge needle at 1 ml/minute, at room temperature. As indicated in Figure 2, significantly reduced injection force was observed when benzyl alcohol (e.g., formulation 6) is used as the solvent, in contrast to formulations using benzyl benzoate (e.g., formulation 5) and benzyl benzoate with ethanol (e.g., formulation 7) as a thixotropic agent, respectively. Notably, due to the shear thing behavior, the formulations using benzyl alcohol as the solvent (e.g., formulation 6), and benzyl benzoate with ethanol as a thixotropic agent (e.g., formulation 7) showed significantly reduced injection force while maintaining viscosities equal to or greater than the formulations using benzyl benzoate (e.g., formulation 5), at lower shear rate; thus maintaining the intactness of the depot after injection into the animals.

EXAMPLE 4

The injection force required to dispense depot vehicles was evaluated for a series of vehicles. Formulations containing PLGA RG502 at various weight percents were each combined with solvents as follows: 100% benzyl benzoate; 75 wt.% benzyl benzoate, 25 wt.% benzyl alcohol; and 100% benzyl alcohol. The amount of the solvent was added to bring the total amount of the formulation to 100%, e.g., if PLGA-502 was used at 45 wt.%, 55 wt.% of the solvent was used. The formulations were then tested for the injection force necessary to pass the formulation through a 24-gauge needle at 1 ml/minute, at room temperature. As seen in Figure 3, benzyl alcohol offers flexibility for depot vehicle formulation, thereby enabling the formulation of depot vehicles with much higher PLGA molecular weights while maintaining reasonably low injection force as compared to similar benzoyl benzoate-containing formulations. Furthermore, for any given percentage of PLGA-502 in the formulation, the injection force decreases as the percentage of the benzyl alcohol increases, as illustrated in Figure 4.

Example 5

hGH Particle Preparation

Human growth hormone (hGH) particles (optionally containing zinc acetate) were prepared as follows:
hGH solution (5 mg/ml) solution in water (BresaGen Corporation, Adelaide, Australia) was concentrated to 10 mg/mL using a Concentration/ Dialysis Selector diafiltering apparatus. The diafiltered hGH solution was washed with 5 times volume of tris or phosphate buffer solution (pH 7.6). Particles of hGH were then formed by spray drying or lyophilization using conventional techniques. Phosphate buffer solutions (5 or 50 mM) containing hGH (5 mg/mL) (and optionally various levels of zinc acetate (0 to 30 mM) when Zn complexed particles were prepared) were spray-dried using a Yamato Mini Spray dryer set at the following parameters:

| Spray Dryer Parameter | Setting |
|---|---|
| Atomizing Air | 2 psi |

(continued)

| Spray Dryer Parameter | Setting |
|---|---|
| Inlet Temperature | 120°C |
| Aspirator Dial | 7.5 |
| Solution Pump | 2-4 |
| Main Air Valve | 40-45 psi |

Lyophilized particles were prepared from tris buffer solutions (5 or 50 mM: pH 7.6) containing hGH (5 mg/mL) using a Durastop µP Lyophilizer in accordance with the following freezing and drying cycles:

| | |
|---|---|
| Freezing cycle | Ramp down at 2.5 C/min to -30° C and hold for 30 min |
| | Ramp down at 2.5 C/min to -30° C and hold for 30 min |
| Drying cycle | Ramp up at 0.5 C/min to 10° C and hold for 960 min |
| | Ramp up at 0.5 C/min to 20° C and hold for 480 min |
| | Ramp up at 0.5 C/min to 25° C and hold for 300 min |
| | Ramp up at 0.5 C/min to 30° C and hold for 300 min |
| | Ramp up at 0.5 C/min to 5° C and hold for 5000 min |

Example 6

HGH-Stearic Acid Particle Preparation

Human growth hormone (hGH) particles were prepared as follows: Lyophilized hGH (3.22 grams, Pharmacia-Upjohn, Stockholm, Sweden) and stearic acid (3.22 grams, 95% pure, Sigma-Aldrich Corporation, St. Louis, MO) were blended and ground. The ground material was compressed in a 13 mm round die, with a force of 44,500 N (10,000 pounds) for 5 minutes. Compressed tablets were ground and sieved through a 70 mesh screen followed by a 400 mesh screen to obtain particles having a size range between 38 - 212 microns.

Example 7

Bupivacaine-Stearic Acid Particle Preparation

Bupivacaine particles were prepared as follows: Bupivacaine hydrochloride (100 grams, Sigma-Aldrich Corporation, St. Louis, MO) was and sieved through 63 -125 micron sieves. The bupivacaine particles and stearic acid (100 grams, 95% pure, Sigma-Aldrich Corporation, St. Louis, MO) were blended and ground. The ground material was compressed in a 13 mm round die, with a force of 5,000 pounds for 5 minutes. Compressed tablets were ground and sieved through a 120 mesh screen followed by a 230 mesh screen to obtain particles having a size range between 63 - 125 microns.

Examples 8

Drug Loading

Compressed particles comprising beneficial agent/stearic acid prepared as above are added to a gel vehicle in an amount of 10 - 20 % by weight and blended manually until the dry powder is wetted completely. Then, the milky light yellow particle/gel mixture is thoroughly blended by conventional mixing using a Caframo mechanical stirrer with an attached square-tip metal spatula. Resulting formulations are illustrated in Table 2 below. Final homogenous gel formulations were transferred to 3, 10 or 30 ml disposable syringes for storage or dispensing.

Table 2

| Formulation | Polymer (%) | Benzyl Benzoate (%) | Benzyl Alcohol (%) | Ethanol (%) |
|---|---|---|---|---|
| 8[a] | 45.0[1] | 45.0 | 0.0 | 0.0 |
| 9[a] | 39.6[1] | 49.5 | 0.0 | 0.9 |
| 10[a] | 45.0[1] | 33.8 | 11.3 | 0.0 |
| 11[a] | 45.0[2] | 33.8 | 11.3 | 0.0 |
| 12[b] | 58.5[3] | 31.5 | 0.0 | 0.0 |
| 13[b] | 58.5[3] | 0.0 | 31.5 | 0.0 |
| 14[b] | 67.5[3] | 0.0 | 22.5 | 0.0 |
| 15[b] | 67.5[4] | 0.0 | 22.5 | 0.0 |
| 16[c] | 60.0[4] | 0.0 | 20.0 | 0.0 |

1 = PLGA RG502 polymer (MW 16,000);
2 = PLGA UG 50/50 (MW 22,600);
3 = PLGA UG 50/50 (MW 8,000);
4 = PLGA L/G 50/50 (MW 10,000);
a = 5% hGH, 5% SA;
b = 10% bupivacaine;
c = 10% bupivacaine, 10% SA.

A representative number of implantable gels were prepared in accordance with the foregoing procedures and tested for in vitro release of beneficial agent as a function of time and also in in vivo studies in rats to determine release of the beneficial agent as determined by blood serum concentrations of beneficial agent as a function of time.

Example 9

hGH In Vivo Studies

In vivo studies in rats were performed following an open protocol to determine serum levels of hGH upon systemic administration of hGH via the implant systems of this invention. Depot gel hGH formulations were loaded into customized 0.5 ml disposable syringes. Disposable 16 gauge needles were attached to the syringes and were heated to 37°C using a circulator bath. Depot gel hGH formulations were injected into immunosuppressed rats and blood was drawn at specified time intervals. All serum samples were stored at 4°C prior to analysis. Samples were analyzed for intact hGH content using a radio immuno assay (RIA). At the end of study the rats are euthanized for gross clinical observation and the depot was retrieved for intactness observations.

Figures 5 & 6 illustrate representative in vivo release profiles of human growth hormone ("hGH") obtained in rats from various depot formulations, including those of the present invention. The in vivo release profile of the depot formulations with benzyl alcohol (e.g., formulations 10 and 11) are comparable to the control formulations (without benzyl alcohol, e.g. formulations 8 and 9). Thus, the depot formulations of the present invention reduce the injection force significantly without compromising the in vivo release profile of the beneficial agent.

At the end of study (i.e. at day 28) the depots were retrieved from the rats. Generally, a one-piece intact round-shaped depot was recovered corresponding to each injected depot in the animal.

Example 10

Bupivacaine In Vivo Studies

In vivo studies in rats (4 per group) were performed following an open protocol to determine plasma levels of bupivacaine upon systemic administration of bupivicaine via the implant systems of this invention. Depot gel bupivacaine formulations were loaded into customized 0.5 ml disposable syringes. Disposable 18 gauge needles were attached to the syringes and were heated to 37°C using a circulator bath. Depot gel bupivacaine formulations were injected into rats and blood was drawn at specified time intervals (1 hour, 4 hours and on days 1, 2, 5, 7, 9 and 14) and analyzed for bupivacaine using LC/MS. At the end of study (i.e., at day 14) the rats were euthanized for gross clinical observation

and the depot was retrieved for intactness observations.

Figures 7, 8 & 9 illustrate representative in vivo release profiles of bupivacaine obtained in rats from various depot formulation, including those of the present invention. The in vivo release profile of the depot formulations with benzyl alcohol (e.g. formulations 13-16) are comparable to the control formulations (without benzyl alcohol, e.g. formulation 12). Thus, the depot formulations of the present invention reduce the injection force significantly without compromising the in vivo release profile of the beneficial agent.

At the end of study (i.e. at day 14) the depots were retrieved from the rats. Generally, a one-piece intact round-shaped depot was recovered corresponding to each injected depot in the animal.

Example 11

Stability of hGH in the depot formulations

Depot gel hGH formulations were stored at 5 °C. At predetermined time points, the depot gel hGH formulation (0.3 ml) was treated with a cooled organic solvent (a 50/50 mixture of methylene chloride/acetone, 5°C, 3x3 ml) to extract the polymer and solvents from the depot formulation. The resulting residual hGH was dissolved in a PBS buffer (2 ml, pH 7.4) and the purity of the hGH was analyzed by size exclusion chromatography (SEC). Figure 10 illustrates the stability of hGH in the various depot gel hGH formulations, including those of the present invention, as a function of time at 5 °C. The stability of hGH in the depot formulations comprising benzyl alcohol is comparable to the control formulations without benzyl alcohol. Thus, the depot formulations of the present invention reduce the injection force significantly without compromising the stability of the beneficial agent, e.g. hGH.

Example 12

Parameters affecting the injection force

$$F = 0.028 \bullet \frac{r^{2.475} \bullet L^{0.770} \bullet Q^{0.716}}{R^{2.630}}$$

The following parameters affect the injection force for a given formulation at pre-set temperature: the radius of syringe (r); inner radius of needle (R); needle length (L); injection speed (Q). The effect of these four parameters on the injection force was determined using a fractional factorial design approach (8 trials) with one near center point for confirmation. The details of the design are summarized in Table 3 (trials 1-9). The injection force was tested using the following formulation (n = 3): the vehicle containing PLGA RG502/BB/BA (40/45/15 wt%), loaded with lysozyme particles (10 wt% 30 μm). The correlation between the injection force and testing parameters was established using JMP software (which is very similar to the Power Law prediction) as follows:

Table 3

| Trial | Needle ID [a] (mm) | Needle length [b] (mm) | Syringe ID[c] (mm) | Injection speed (mL/min) | Injection | Force (N) |
|---|---|---|---|---|---|---|
| | | | | | Avg | SD |
| 1 | 0.191 | 12.7 | 2.3 | 0.05 | 14.6 | 0.8 |
| 2 | 0.292 | 50.8 | 3.25 | 0.5 | 172.2 | 5.3 |
| 3 | 0.292 | 12.7 | 3.25 | 0.05 | 8.6 | 0.2 |
| 4 | 0.191 | 12.7 | 3.25 | 0.5 | 176.0 | 2.6 |
| 5 | 0.292 | 50.8 | 2.3 | 0.05 | 13.4 | 0.3 |
| 6 | 0.292 | 12.7 | 2.3 | 0.5 | 30.0 | 2.5 |
| 7 | 0.191 | 50.8 | 3.25 | 0.05 | 127.0 | 2.3 |

(continued)

| Trial | Needle ID [a] (mm) | Needle length [b] (mm) | Syringe ID[c] (mm) | Injection speed (mL/min) | Injection Force (N) Avg | SD |
|---|---|---|---|---|---|---|
| 8 | 0.191 | 50.8 | 2.3 | 0.5 | 161.4 | 4.5 |
| 9 | 0.241 | 25.4 | 2.3 | 0.25 | 48.8 | 0.5 |

[a] Needles having following gauges were used: 24G (ID = 0.292 mm), 25G (ID = 0.241 mm) and 27G (ID = 0.191 mm);
[b] Needle having following lengths were used: 0.5 inch (12.7 mm), 1 inch (25.4 mm), 2 inches (50.8 mm);
[c] Two different syringes (Hamilton): 250 µL (ID = 2.30 mm); 500 µL (ID = 3.25 mm).

Example 13

Effect of drug particle size and loading on the injection force of depot formulations

Particle size and amount of loading of the beneficial agent, i.e. drug, are additional factors potentially affecting the injection force of the depot formulation. Depot gel lysozyme formulations were used to determine the effect of drug particle size and loading on the injection force of the depot formulations. Various depot gel lysozyme formulations of present invention containing differing amounts (5-30% loading) and particle sizes (5-50 µm) of lysozyme were tested for injection force using 27 gauge, 2" needles. The injection speed was set at 50 µl/min. The formulations tested are summarized in Table 4. As illustrated in Figure 11, the injection force of the depot formulations increases with the increase of drug particle loading. With 10 wt% particle loading, the injection forces increase about 50% compared to the corresponding gel formulation, regardless of the composition of the gel formulation. The injection force appears to be proportional to the amount of benzyl alcohol in the gel formulation, further indicating that benzyl alcohol significantly reduces the injection force of the depot gel formulations of the invention.

Table 4

| Formulation | PLGA RG 502 (wt%) | Benzyl Benzoate (BB, wt%) | Benzyl Alcohol (BA, wt%) | Particle loading (wt%) | Particle size (µm) |
|---|---|---|---|---|---|
| 18 | 38.0 | 42.8 | 14.2 | 5 | 5 |
| 19 | 34.0 | 38.3 | 12.8 | 15 | 5 |
| 20 | 38.0 | 42.8 | 14.2 | 5 | 50 |
| 21 | 34.0 | 38.3 | 12.8 | 15 | 50 |
| 22 | 36.0 | 40.5 | 13.5 | 10 | 20 |
| 23 | 38.0 | - | 57.0 | 5 | 5 |
| 24 | 34.0 | - | 51.0 | 15 | 5 |
| 25 | 38.0 | - | 57.0 | 5 | 50 |
| 26 | 34.0 | - | 51.0 | 15 | 50 |
| 27 | 36.0 | - | 54.0 | 10 | 20 |
| 28 | 30.8 | 34.7 | 11.6 | 23 | 50 |
| 29 | 28.0 | 31.5 | 10.5 | 30 | 50 |
| 30 | 30.8 | - | 46.2 | 23 | 50 |
| 31 | 28.0 | - | 42.0 | 30 | 50 |
| 32 | 40.0 | 45.0 | 15.0 | 0 | - |
| 33 | 40.0 | - | 60.0 | 0 | - |

Example 14

PDGF Preformulation Preparation

Various Platelet Derived Growth Factor (PDGF) preformulations were prepared as follows:

Dialysis

The following buffers were prepared for the dialysis:

(A) The histidine buffer (10 mM, pH 6, 2 L) was prepared as follows. L-histidine (3.10g) was weighed in a volumetric flask (2 L). Milli-Q water (1800m)) was added to the flask and the mixture was stirred until the solid dissolved. HCl (0.1 N, 8 ml) was added, the pH was checked and adjusted to 6. The solution was diluted with milli-Q water to a volume of 2L.
(B) The succinate buffer (10mM, pH 6, 2 L) was prepared as follows. Succinic acid (5.91 g) was weighed in a volumetric flask (250ml) and milli-Q water (250ml) was added to obtain succinic acid solution (0.2M). NaOH solution (4g, 50% w/w) was measured in a volumetric flask (250ml) and diluted with milli-Q water to obtain NaOH solution (0.2M). The succinic acid solution (0.2M, 100ml) was mixed with the NaOH solution (0.2M, 165ml) and milli-Q water (1600ml) in a volumetric flask (2L) the pH was checked and adjusted to 6. The solution was diluted with milli-Q water to a volume of 2L.

The PDGF-BB bulk solution, i.e. aqueous solution of PDGF in acetate buffer, was thawed to room temperature. Various aliquots of the PDGF-BB solution were diluted appropriately for a UV absorbance measurement, using a 1 cm path length cuvette from 400 to 250 nm. The absorbance was recorded at 280 nm and corrected for light scattering in the 400 to 330 nm range using a log(Absorbance) vs. log(wavelength) extrapolation. The concentration of PDGF-BB was determined using an extinction coefficient of 0.574 ml/mg x cm. The PDGF-BB solution was concentrated using a Millipore Tangential Flow Filtration System (having a reservoir (100 ml) and a Pellicon XL PLCCC 5000 MWCO regenerated cellulose membrane), and the protein was divided into two parts. One half of the protein was diafiltered against the histidine buffer (10mM, pH 6); and the second half of the protein was diafiltered against the succinate buffer (10mM, pH 6), according to manufacturer's instructions. After diafiltration, an aliquot from each part was appropriately diluted for an absorbance measurement as described above, and analyzed by reverse phase and size exclusion high pressure liquid chromatography (HPLC). The protein solution was removed from the TFF system according to Millipore TFF instructions.

PDGF-BB pre-formulation

Various pre-formulations of PDGF-BB were prepared by adding different excipients, e.g. sucrose, tween 20, Zn acetate or combinations thereof, into the above diafiltrated PDGF-BB solution; the solution was buffered either with histidine or succinate to obtain the final PDGF-BB concentration in the solution of approximately 5 mg/ml (as tabulated in Tables 5 and 6). Those solutions were lyophilized under the conditions described below to achieve the dry PDGF-BB formulations.

Lyophilization

The lyophilization freezing cycle was started with an equilibration of shelf temperature at 4°C at 2.5°C/min and held at this temperature for 30 minutes. The temperature was then brought down to - 50°C at 2.5°C/min and held for 3 hours. For the primary drying cycle, vacuum was applied and the shelf temperature was increased as follows: (i) -20°C at 0.14°C/min for 24 hours; (ii) -15°C at 0.14°C/min for 24 hours; and (iii) 0°C at 0.14°C/min for 12 hours. For the secondary drying cycle involves the shelf temperature was increased as follows: (i) 20°C at 0.14°C/min for 12 hours; and (ii) 30°C at 0.14°C/min for 4 hours. After drying, shelf temperature was decreased to 0°C or 4°C and held at that temperature until removal from the instrument. The vials were capped using shelf stoppering the run was stopped and the vials were removed.

Example 15

Preliminary stability of PDGF preformulations in the gel vehicle

All lyophilized protein formulation as listed in Tables 5 and 6, were mixed into a gel vehicle with the composition

of PLGA RG502/Benzyl Benzoate (BB)/ benzyl alcohol (BA) of 40/45/15 with the loading of the protein formulation about 10 wt%. After stored at 5 °C for 1 day, the mixtures were extracted with a organic solvent mixture of methylene chloride and acetone (ratio of 50/50) as described in the example 15 above. The purity of the PDGF-BB was analyzed by both reverse phase HPLC (rpHPLC) and size exclusion chromatography (SEC). The stability data of the PDGF-BB formulation after mixing with the gel vehicle are summarized in Tables 5 and 6. In general, no distinguishable degradation of the PDGF-BB was found in the PDGF-BB formulation incorporated with the excipients as described in the Example14 and mixed with the gel vehicle of the present invention.

Table 5

| Formulation | SA-1 | SA-2 | SA-3 | SA-4 | SA-5 | Bulk PDGF |
|---|---|---|---|---|---|---|
| PDGF (mg) | 1 | 1 | 1 | 1 | 1 | |
| Sucrose (mg) | 1 | 1 | 0 | 0 | 0 | |
| Tween 20 (mg) | 0 | 0.2 | 0.2 | 0 | 0 | |
| Succinate (mg) | 0.24 | 0.24 | 0.24 | 0.24 | 0.24 | |
| Zn acetate (mg) | 0 | 0 | 0 | 0 | 0.02 | |
| Gel vehicle (mg)[a] | 20.16 | 21.96 | 12.96 | 11.16 | 11.34 | |
| % PDGF monomer by SEC | 98.90 | 98.82 | 98.02 | 98.51 | 98.59 | 99.27 |
| % PDGF dimmer by SEC | 1.10 | 1.18 | 1.98 | 1.49 | 1.41 | 0.73 |
| % peak at RRT = 0.93 by rp-HPLC | 11.5 | 11.1 | 10.7 | 12.7 | 11.0 | 11.1 |
| % peak at RRT = 1.00 by rp-HPLC | 87.3 | 87.6 | 87.6 | 86.2 | 87.8 | 87.7 |
| % peak at RRT = 1.10 by rp-HPLC | 1.1 | 1.2 | 1.1 | 1.1 | 1.1 | 1.2 |
| % other peaks by rp-HPLC | 0.0 | 0.1 | 0.6 | 0.0 | 0.0 | 0.0 |
| a = PLGA RG502/BB/BA - 40/45/15 | | | | | | |

Table 6

| Formulation | HA-1 | HA-2 | HA-3 | HA-4 | HA-5 | Bulk PDGF |
|---|---|---|---|---|---|---|
| PDGF (mg) | 1 | 1 | 1 | 1 | 1 | |
| Sucrose (mg) | 1 | 1 | 0 | 0 | 0 | |
| Tween 20 (mg) | 0 | 0.2 | 0.2 | 0 | 0 | |
| Histidine (mg) | 0.31 | 0.31 | 0.31 | 0.31 | 0.31 | |
| Zn acetate (mg) | 0 | 0 | 0 | 0 | 0.02 | |
| Gel vehicle (mg)[a] | 20.79 | 22.59 | 13.59 | 11.79 | 11.97 | |
| % PDGF monomer by SEC | 99.15 | 99.15 | 99.07 | 99.01 | 99.04 | 99.27 |
| % PDGF dimer by SEC | 0.85 | 0.85 | 0.93 | 0.99 | 0.96 | 0.73 |
| % peak at RRT = 0.93 by rp-HPLC | 11.3 | 11.0 | 10.9 | 10.8 | 10.9 | 11.1 |
| % peak at RRT = 1.00 by rp-HPLC | 87.6 | 87.8 | 87.7 | 88.0 | 88.0 | 87.7 |
| % peak at RRT = 1.10 by rp-HPLC | 1.1 | 1.1 | 1.2 | 1.2 | 1.1 | 1.2 |
| % other peaks by rp-HPLC | 0.0 | 0.0 | 0.2 | 0.0 | 0.0 | 0.0 |
| a = PLGA RG502/BB/BA - 40/45/15 | | | | | | |

Example 16

Preparation of PDGF Particles

PDGF-BB formulations with sucrose in histidine buffer and without sucrose in succinate buffer were prepared as similar way to the Example 14 above (Table 7): Thaw PDGF-BB bulk solution. Combine the solution and measure volume in a graduate cylinder. Take an aliquot and dilute appropriately for a UV absorbance measurement. Record the absorbance in a 1 cm path length cuvette from 400 to 250 nm. Record the absorbance at 280 nm and correct for light scattering in the 400 to 330 nm range using a log(Absorbance) vs. log(wavelength) extrapolation. Determine the concentration of PDGF-BB using an extinction coefficient of 0.574 ml/mg x cm. Using a Millipore Tangential Flow Filtration System with 100 ml reservoir and a Pellicon XL PLCCC 5000 MWCO regenerated cellulose membrane, concentrate if necessary, and diafilter half of the protein against 10mM histidine pH 6 and concentrate, if necessary, and diafilter the other half against 10mM succinate pH 6, according to TFF instructions. After diafiltration, remove an aliquot from each and dilute appropriately for a UV absorbance measurement and analyze by reverse phase and size exclusion HPLC. Remove all of the protein solution from the TFF system according to Millipore TFF instructions. For PDGF-BB in 10mM histidine add sucrose to give a 1:1 final ratio with the protein (PDGF-BB at a final concentration of ~5 mg/ml). For the PDGF-BB in 10mM succinate pH 6 dilute with 10mM succinate to give a final protein concentration of approximately 5 mg/ml. Aliquot formulations were placed into glass lyophilization vials and were lyophilized under the conditions described in the Example 18 to achieve the lyophilized dry PDGF-BB formulations. Lyophilized PDGF formulations were ground in an agate mortar and pestle. The grounded particles were sieved through a US #230 Mesh Screen (63 μm) and are collected on a US #500 Mesh Screen (25 μm).

Table 7

| Formulation | PDGF-BB (wt%) | Succinate (wt%) | Histidine (wt%) | Sucrose (wt%) |
|---|---|---|---|---|
| 34 | 81 | 19 | - | |
| 35 | 43 | - | 14 | 43 |

Example 17

Preparation of PDGF depot formulations

The PDGF depot formulations were prepared in two steps. The first step was to make the gel formulations using the procedure as described below. Appropriate amounts of pre-irradiated PLGA RG 502 and solvent were dispensed into the Keyence hybrid mixer bowl (made from high density polyethylene (HDPE)). The mixing bowl was tightly sealed, placed into the hybrid mixer (Model HM-501, Keyence Corp., Japan) and mixed (5-10 minutes) at the mixing speed (revolution 2000 rpm, rotation 800 rpm).

Mixing of particles in the gel was performed at room temperature in a glass syringe (10 ml or 25 ml). The PDGF particles and gel were first weighed and transferred into the syringe. Then, the PDGF particles and gel mixture were thoroughly blended by conventional mixing using a Caframo mechanical stirrer with an attached square-tip metal spatula. Resulting formulations are tabulated in Table 8.

Table 8

| Formulation | Polymer (%) (PLGA RG502, MW = 16,000) | Benzyl Benzoate (%) | Benzyl Alcohol (%) |
|---|---|---|---|
| 36[a] | 31.5 | 43.9 | 14.6 |
| 37[b] | 31.5 | 43.9 | 14.6 |
| 38[a] | 31.5 | 29.3 | 29.2 |
| 39[b] | 31.5 | 29.3 | 29.2 |
| a = 10% formulation 34;<br>b = 10% formulation 35. | | | |

Example 18

Stability of PDGF in the depot formulations

Depot gel PDGF formulations were stored for different periods of time at 5, 25 and 40 °C, respectively. At predetermined time points, the depot gel PDGH formulation (0.3 ml) was treated with a cooled organic solvent (a ' 50/50 mixture of methylene chloride/acetone, at 5°C, 3x3.0 ml). The resulting residual PDGF was dissolved in a PBS buffer (2 ml, pH 7.4) and the purity of the PDGF was analyzed by both reverse phase HPLC (rpHPLC) and size exclusion chromatography (SEC) HPLC. Figures 12-14 illustrate the stability of PDGF in the various depot formulations, including those of the present invention, as a function of time at 5 °C (Figure 12), 25 °C (Figure 13) and 40 °C (Figure 14), respectively. Table 9 summarizes the chemical stability of PDGF tested by rpHPLC in the various depot formulations, including those of the present invention, as a function of time at 5 °C, 25 °C and 40 °C, respectively. As illustrated in Figures 12-14, depot gel PDGF formulations containing sucrose demonstrated surprisingly stability with minimal lose of monomer content, as compared to the depot gel PDGF formulations without sucrose, at all temperatures measured.

Table 9

| | | | | RP-HPLC (% Peak Area) | | |
|---|---|---|---|---|---|---|
| Formulation | Temp. | Time (day) | Peak at (RRT=0.93) | Peak at (RRT=1.00) | Peak at (RRT=1.09) | Other Peak(s) |
| Bulk PDGF | | 0 | 11.1 | 87.7 | 1.2 | 0 |
| | | 0 | 13.03 ± 0.12 | 85.04 ± 0.43 | 1.2 ± 0.35 | 0.72 ± 0.09 |
| | 5°C | 14 | 12.77 ± 0.28 | 85.94 ± 0.17 | 1.06 ± 0.03 | 0.23 ± 0.19 |
| | 5°C | 28 | 12.17 ± 0.32 | 86.03 ± 0.77 | 1.11 ± 0.34 | 0.69 ± 0.08 |
| | 5°C | 90 | 12.14 ± 0.35 | 86.14 ± 0.42 | 0.78 ± 0.01 | 0.94 ± 0.08 |
| 36 | 25°C | 14 | 9.57 ± 0.14 | 89.52 ± 0.18 | (shoulder) | 0.91 ± 0.03 |
| | 25°C | 28 | 8.24 ± 0.12 | 90.98 ± 0.09 | (shoulder) | 0.78 ± 0.04 |
| | 25°C | 90 | 8.96 ± 0.21 | 90.16 ± 0.23 | (N/A) | 0.88 ± 0.01 |
| | 40°C | 14 | 7.22 ± 0.06 | 91.96 ± 0.09 | (shoulder) | 0.83 ± 0.02 |
| | 40°C | 28 | 5.54 ± 0.13 | 93.80 ± 0.09 | (shoulder) | 0.66 ± 0.09 |
| | | | | | | |
| | | 0 | 13.25 ± 0.16 | 84.97 ± 0.34 | 1.5 ± 0.36 | 0.28 ± 0.86 |
| | 5°C | 14 | 13.07 ± 0.04 | 85.32 ± 0.34 | 1.43 ± 0.36 | 0.18 ± 0.03 |
| | 5°C | 28 | 12.93 ± 0.08 | 85.62 ± 0.43 | 1.27 ± 0.37 | 0.18 ± 0.06 |
| | 5°C | 90 | 14.07 ± 0.25 | 83.87 ± 0.41 | 1.39 ± 0.44 | 0.67 ± 0.28 |
| 37 | 25°C | 14 | 12.19 ± 0.10 | 86.28 ± 0.52 | 1.25 ± 0.33 | 0.28 ± 0.13 |
| | 25°C | 28 | 11.79 ± 0.27 | 86.82 ± 0.09 | 1.30 ± 0.35 | 0.10 ± 0.02 |
| | 25°C | 90 | 14.57 ± 0.11 | 83.84 ± 0.57 | 1.43 ± 0.46 | 0.17 ± 0.00 |
| | 40°C | 14 | 12-93 ± 0.08 | 85.65 ± 0.26 | 1.26 ± 0.39 | 0.16 ± 0.07 |
| | 40°C | 28 | 13.09 ± 0.24 | 85.18 ± 0.17 | 1.59 ± 0.43 | 0.15 ± 0.04 |
| | | | | | | |
| | | 0 | 12.39 ± 0.28 | 85.91 ± 0.26 | 0.96 ± 0.02 | 0.73 ± 0.04 |
| | 5°C | 14 | 12.21 ± 0.29 | 86.05 ± 0.34 | 1.10 ± 0.32 | 0.64 ± 0.36 |
| | 5°C | 28 | 11.38 ± 0.18 | 87.11 ± 0.70 | 0.81 ± 0.04 | 0.97 ± 0.08 |
| 38 | 25°C | 14 | 8.50 ± 0.19 | 90.40 ± 0.27 | (shoulder) | 1.10 ± 0.08 |
| | 25°C | 28 | 7.73 ± 0.19 | 91.25 ± 0.18 | (shoulder) | 1.02 ± 0.04 |

(continued)

| | | | | RP-HPLC (% Peak Area) | | |
|---|---|---|---|---|---|---|
| Formulation | Temp. | Time (day) | Peak at (RRT=0.93) | Peak at (RRT=1.00) | Peak at (RRT=1.09) | Other Peak(s) |
| | 25°C | 90 | 7.48 ± 0.64 | 91.67 ± 0.66 | (N/A) | 0.86 ± 0.01 |
| | 40°C | 14 | (shoulder) | 99.17 ± 0.00 | (shoulder) | 0.83 ± 0.04 |
| | 40°C | 28 | (shoulder) | 99.56 ± 0.00 | (shoulder) | 0.44 ± 0.03 |
| | | | | | | |
| | | 0 | 12.71 ± 0.14 | 85.90 ± 0.26 | 1.1 ± 0.01 | 0.3 ± 0.03 |
| | 5°C | 14 | 13.04 ± 0.25 | 85.10 ± 0.60 | 1.45 ± 0.37 | 0.41 ± 0.13 |
| | 5°C | 28 | 12.67 ± 0.20 | 86.05 ± 0.17 | 1.04 ± 0.02 | 0.24 ± 0.05 |
| | 5°C | 90 | 14.65 ± 0.08 | 83.65 ± 0.07 | 1.04 ± 0.01 | 0.66 ± 0.13 |
| 39 | 25°C | 14 | 12.94 ± 0.06 | 85.27 ± 0.43 | 1.50 ± 0.33 | 0.29 ± 0.10 |
| | 25°C | 28 | 12.64 ± 0.19 | 85.55 ± 0.34 | 1.51 ± 0.41 | 0.30 ± 0.09 |
| | 25°C | 90 | 14.11 ± 0.15 | 84.68 ± 0.10 | 1.01 ± 0.01 | 0.21 ± 0.04 |
| | 40°C | 14 | 12.10 ± 0.18 | 85.76 ± 0.34 | 1.26 ± 0.39 | 0.87 ± 0.46 |
| | 40°C | 28 | 11.12 ± 0.22 | 88.05 ± 0.88 | (shoulder) | 0.19 ± 0.03 |

Example 19

In vitro release of PDGF from the depot formulations

The in vitro release of PDGF from the depot gel PDGF formulation of the present Invention was performed as follows. The depot gel PDGF formulation (80-120 mg) was loaded into a tea bag and placed in a 20 mL scintillation vial and the release medium (5 mL, phosphate buffer saline (PBS) + 0.1% Tween 20, pH 7.4) was added to the vial. The vial was incubated In a 37 °C water bath with gentle agitation. The medium was replaced daily for the first 5 days, then twice a week thereafter till the end of release duration. The amount of PDGF released from the depot was measured by size exclusion chromatography (SEC) HPLC. AS illustrated in Figure 15, sustained release of PDGF from the depot formulations of the present invention was obtained for over a month.

## Claims

1. A composition comprising: a bioerodible, biocompatible polymer in an amount of from 35 wt. % to 75 wt. % of the composition; an aromatic alcohol having miscibility in water of less than or equal to 7 wt. % at 25 °C, in an amount effective to plasticize the polymer and form a gel therewith; and a beneficial agent in the form of particles, wherein the composition is free of monohydric C1-C6 alkanols for use in a method of treatment by injection into a patient's body to form an implant.

2. The composition of claim 1, wherein the aromatic alcohol has the structural formula (I)

$$Ar\text{- } (L)\ n\text{-}OH\ (I) \qquad (I)$$

wherein Ar is aryl or heteroaryl, n is zero or 1, and L is a linking moiety.

3. The composition of claim 2, wherein Ar is monocyclic aryl or heteroaryl, n is 1, and L is C1-C6 alkylene optionally containing at least one heteroatom.

4. The composition of claim 3, wherein Ar is monocyclic aryl and L is C1-C6 alkylene.

5. The composition of claim 4, wherein Ar is phenyl and L is methylene.

6. The composition of claim 1, wherein the polymer is selected from the group consisting of polylactides, polyglycolides, polycaprolactones, polyanhydrides, polyamines, polyurethanes, polyesteramides, polyorthoesters, polydioxanones, polyacetals, polyketals, polycarbonates, polyphosphoesters, polyorthocarbonates, polyphosphazenes, succinates, poly (malic acid), poly (amino acids), polyvinylpyrrolidone, polyethylene glycol, polyhydroxycellulose, chitin, chitosan, hyaluronic acid, and copolymers, terpolymers and mixtures thereof.

7. The composition of claim 1, wherein the polymer is a lactic acid-based polymer.

8. The composition of claim 7, wherein the polymer is a copolymer of lactic acid and glycolic acid.

9. The composition of claim 2 wherein the bioerodible, biocompatible polymer is a biodegradable, biocompatible lactic acid-based polymer having a weight average molecular weight in the range of 5,000 to 50,000.

10. The composition of claim 9, wherein the polymer is a copolymer of lactic acid and glycolic acid.

11. The composition of claim 10, wherein the aromatic alcohol is benzyl alcohol.

12. The composition of any of the preceding claims, wherein the aromatic alcohol has miscibility in water of less than or equal to 5 wt. % at 25 °C.

13. The composition of any of the preceding claims, wherein the aromatic alcohol has miscibility in water of less than or equal to 3 wt. % at 25 °C.

14. The composition of any of the preceding claims, wherein the aromatic alcohol has miscibility in water of less than or equal to 1 wt. % at 25 °C.

15. The composition of any of the preceding claims, wherein the aromatic alcohol has miscibility in water of less than or equal to 0.5 wt. % at 25 °C.

16. The composition of any of the preceding claims, further including at least one of the following: a pore former; a solubility modulator for the beneficial agent; and an osmotic agent.

17. The composition of any of the preceding claims, wherein the composition is free of solvents having miscibility in water that is greater than 7 wt. % at 25 °C.

18. The composition of any of the preceding claims wherein the beneficial agent is selected from a drug, proteins, enzymes, hormones, polynucleotides, nucleoproteins, polysaccharides, glycoproteins, lipoproteins, polypeptides, steroids, analgesics, local anesthetics, antibiotic agents, chemotherapeutic agents, immunosuppressive agents, anti-inflammatory agents, antiproliferative agents, antimitotic agents, angiogenic agents, anticoagulants, fibrinolytic agents, growth factors, antibodies, ocular drugs, and metabolites, analogs, derivatives, and fragments thereof.

19. The composition of claim 18 wherein the beneficial agent is present in an amount of from 0.1 to 50% by weight of the combined amounts of the polymer, the solvent and the beneficial agent.

20. The composition of claim 18 wherein the particle further comprises a component selected from the group consisting of a stabilizing agent, bulking agent, chelating agent and a buffering agent.

## Patentansprüche

1. Zusammensetzung, umfassend: ein bioerodierbares, biokompatibles Polymer in einer Menge von 35 Gew.-% bis 75 Gew.-% der Zusammensetzung, einen aromatischen Alkohol mit einer Mischbarkeit in Wasser von weniger oder gleich 7 Gew.-% bei 25 °C in einer Menge, die wirksam ist, um das Polymer zu plastifizieren und ein Gel damit zu bilden; sowie ein vorteilhaftes Agens in der Form von Partikeln, während die Zusammensetzung frei ist von monohydrischen C1-C6 Alkanolen zur Verwendung in einem Behandlungsverfahren mittels Injektion in einen Patientenkörper, um ein Implantat zu bilden.

2. Die Zusammensetzung gemäß Patentanspruch 1, während der aromatischer Alkohol die strukturelle Formel (I)

Ar- (L) n-OH (I) (I)

aufweist, wobei Ar ein Aryl oder Heteroaryl ist, n ist null oder 1 und L ist ein Bindungsrest.

3. Die Zusammensetzung gemäß Patentanspruch 2, wobei Ar ein monozyklisches Aryl oder Heteroaryl ist, n ist 1 und L ist ein C1-C6 Alkylen, welches optional mindestens ein Heteroatom aufweist.

4. Die Zusammensetzung gemäß Patentanspruch 3, wobei Ar ein monozyklisches Aryl ist und L ist ein C1-C6 Alkylen.

5. Die Zusammensetzung gemäß Patentanspruch 4, wobei Ar ein Phenyl ist und L Methylen ist.

6. Die Zusammensetzung gemäß Patentanspruch 1, wobei das Polymer ausgewählt ist aus der Gruppe bestehend aus: Polylactiden, Polyglykoliden, Polycaprolactonen, Polyanhydriden, Polyaminen, Polyurethanen, Polyesteramiden, Polyorthoestern, Polydioxanonen, Polyacetalen, Polyketalen, Polycarbonaten, Polyphosphoestern, Polyorthocarbonaten, Polyphosphazenen, Succinaten, Poly(Apfelsäure), Poly(Amionsäuren), Polyvinylpyrrolidone, Polyethylenglykol, Polyhydroxycellulose, Chitin, Chitosan, Hyaluronsäure sowie Co-Polymere, TerPolymere und Mischungen davon.

7. Die Zusammensetzung gemäß Patentanspruch 1, wobei das Polymer ein milchsäurebasiertes Polymer ist.

8. Die Zusammensetzung gemäß Patentanspruch 7, wobei das Polymer ein CoPolymer aus Milchsäure und Glykolsäure ist.

9. Die Zusammensetzung gemäß Patentanspruch 2, wobei das bioerodierbare, biokompatible Polymer ein biologisch abbaubares, biokompatibles milchsäurebasiertes Polymer mit einem mittleren Molekulargewicht im Bereich von 5.000 bis 50.000 ist.

10. Die Zusammensetzung gemäß Patentanspruch 9, wobei das Polymer ein CoPolymer aus Milchsäure und Glykolsäure ist.

11. Die Zusammensetzung gemäß Patentanspruch 10, wobei der aromatische Alkohol Benzylalkohol ist.

12. Die Zusammensetzung gemäß einem der vorhergehenden Patentansprüche, wobei der aromatische Alkohol eine Mischbarkeit in Wasser von weniger oder gleich 5 Gew.-% bei 25 °C aufweist.

13. Die Zusammensetzung gemäß einem der vorhergehenden Patentansprüchen, wobei der aromatische Alkohol eine Mischbarkeit in Wasser von weniger oder gleich 3 Gew.-% bei 25 °C aufweist.

14. Die Zusammensetzung gemäß einem der vorhergehenden Patentansprüche, wobei der aromatische Alkohol eine Mischbarkeit in Wasser von weniger oder gleich 1 Gew.-% bei 25 °C aufweist.

15. Die Zusammensetzung gemäß einem der vorhergehenden Patentansprüche, wobei der aromatische Alkohol eine Mischbarkeit in Wasser von weniger oder gleich 0,5 Gew.-% bei 25 °C aufweist.

16. Die Zusammensetzung gemäß einem der vorhergehenden Patentansprüche, weiterhin aufweisend mindestens eines der folgenden: einen Porenbildner, einen Löslichkeitsregler für das vorteilhafte Agens sowie ein osmotisches Agens.

17. Die Zusammensetzung gemäß einem der vorhergehenden Patentansprüche, wobei die Zusammensetzung frei ist von Lösungsmitteln mit einer Mischbarkeit in Wasser, die größer ist als 7 Gew.-% bei 25 °C.

18. Die Zusammensetzung gemäß einem der vorhergehenden Patentansprüche, wobei das vorteilhafte Agens ausgewählt ist aus: einem Medikament, Proteinen, Enzymen, Hormonen, im Polynukleotiden, Nukleoproteinen, Polysacchariden, Glykoproteinen, Lipoproteinen, Polypeptiden, Steroiden, Analgetika, Lokalanästhetika, antibiotischen Agenzien, chemotherapeutischen Agenzien, immunsuppressiven Agenzien, entzündungshemmenden Agenzien, antiproliferativen Agenzien, antimitotischen Agenzien, angiogenen Agenzien, Antikoagulantien, fibrinolytischen

Agenzien, Wachstumsfaktoren, Antikörpern, Augenmedikamenten und Metaboliten, Analoga, Derivaten und Fragmenten davon.

**19.** Die Zusammensetzung gemäß Patentanspruch 18, wobei das vorteilhafte Agens in einer Menge von 0,1 bis 50 Gew.-% der kombinierten Mengen des Polymers, des Lösungsmittels und der vorteilhafte Agens vorhanden ist.

**20.** Die Zusammensetzung gemäß Patentanspruch 18, wobei die Partikel weiterhin eine Komponente umfassen, die ausgewählt ist aus der Gruppe bestehend aus: einer stabilisierenden Agens, quellenden Agens, chelatbildenden Agens und einer puffernden Agens.

**Revendications**

**1.** Composition comprenant : un polymère biocompatible bioérodable en une quantité de 35 % en poids à 75 % en poids de la composition ; un alcool aromatique présentant une miscibilité dans l'eau inférieure ou égale à 7 % en poids à 25 °C, en une quantité suffisante pour plastifier le polymère et former un gel avec celui-ci ; et un agent bénéfique sous la forme de particules, dans laquelle la composition est exempte d'alcanols monohydriques en $C_1$ à $C_6$ pour l'utilisation dans un procédé de traitement par injection dans le corps d'un patient pour former un implant.

**2.** Composition selon la revendication 1, dans laquelle l'alcool aromatique répond à la formule structurale (I)

$$Ar\text{-} (L)\ n\text{-}OH\ (I) \qquad (I)$$

dans laquelle Ar représente un groupe aryle ou hétéroaryle, n vaut zéro ou 1, et L représente un groupe fonctionnel de liaison.

**3.** Composition selon la revendication 2, dans laquelle Ar représente un groupe aryle ou hétéroaryle monocyclique, n vaut 1, et L représente un groupe alkylène en $C_1$ à $C_6$ contenant facultativement au moins un hétéroatome.

**4.** Composition selon la revendication 3, dans laquelle Ar représente un groupe aryle monocyclique et L représente un groupe alkylène en $C_1$ à $C_6$.

**5.** Composition selon la revendication 4, dans laquelle Ar représente un groupe phényle et L représente un groupe méthylène.

**6.** Composition selon la revendication 1, dans laquelle le polymère est choisi dans le groupe constitué par les polylactides, les polyglycolides, les polycaprolactones, les polyanhydrides, les polyamines, les polyuréthanes, les polyesteramides, les polyorthoesters, les polydioxanones, les polyacétals, les polycétals, les polycarbonates, les polyphosphoesters, les polyorthocarbonates, les polyphosphazènes, les succinates, le poly (acide malique), les poly (acides aminés), la polyvinylpyrrolidone, le polyéthylène glycol, la polyhydroxycellulose, la chitine, le chitosane, l'acide hyaluronique et les copolymères, terpolymères et leurs mélanges.

**7.** Composition selon la revendication 1, dans laquelle le polymère est un polymère à base d'acide lactique.

**8.** Composition selon la revendication 7, dans laquelle le polymère est un copolymère d'acide lactique et d'acide glycolique.

**9.** Composition selon la revendication 2 dans laquelle le polymère biocompatible bioérodable est un polymère à base d'acide lactique biocompatible biodégradable ayant un poids moléculaire moyen en poids dans l'intervalle de 5 000 à 50 000.

**10.** Composition selon la revendication 9, dans laquelle le polymère est un copolymère d'acide lactique et d'acide glycolique.

**11.** Composition selon la revendication 10, dans laquelle l'alcool aromatique est un alcool benzylique.

**12.** Composition selon l'une quelconque des revendications précédentes, dans laquelle l'alcool aromatique présente une miscibilité dans l'eau inférieure ou égale à 5 % en poids à 25 °C.

**13.** Composition selon l'une quelconque des revendications précédentes, dans laquelle l'alcool aromatique présente une miscibilité dans l'eau inférieure ou égale à 3 % en poids à 25 °C.

**14.** Composition selon l'une quelconque des revendications précédentes, dans laquelle l'alcool aromatique présente une miscibilité dans l'eau inférieure ou égale à 1 % en poids à 25 °C.

**15.** Composition selon l'une quelconque des revendications précédentes, dans laquelle l'alcool aromatique présente une miscibilité dans l'eau inférieure ou égale à 0,5 % en poids à 25 °C.

**16.** Composition selon l'une quelconque des revendications précédentes, comprenant en outre au moins l'un des composés suivants : un porogène ; un modulateur de solubilité pour l'agent bénéfique ; et un agent osmotique.

**17.** Composition selon l'une quelconque des revendications précédentes, dans laquelle la composition est exempte de solvants présentant une miscibilité dans l'eau qui est supérieure à 7 % en poids à 25 °C.

**18.** Composition selon l'une quelconque des revendications précédentes dans laquelle l'agent bénéfique est choisi parmi un médicament, des protéines, des enzymes, des hormones, des polynucléotides, des nucléoprotéines, des polysaccharides, des glycoprotéines, des lipoprotéines, des polypeptides, des stéroïdes, des analgésiques, des anesthésiques locaux, des agents antibiotiques, des agents chimiothérapeutiques, des agents immunosuppresseurs, des agents anti-inflammatoires, des agents antiprolifératifs, des agents antimitotiques, des agents angiogéniques, des anticoagulants, des agents fibrinolytiques, des facteurs de croissance, des anticorps, des médicaments oculaires, et des métabolites, analogues, dérivés et fragments de ceux-ci.

**19.** Composition selon la revendication 18 dans laquelle l'agent bénéfique est présent en une quantité de 0,1 à 50 % en poids des quantités combinées du polymère, du solvant et de l'agent bénéfique.

**20.** Composition selon la revendication 18 dans laquelle la particule comprend en outre un composant choisi dans le groupe constitué par un stabilisant, un agent de charge, un agent chélatant et agent tampon.

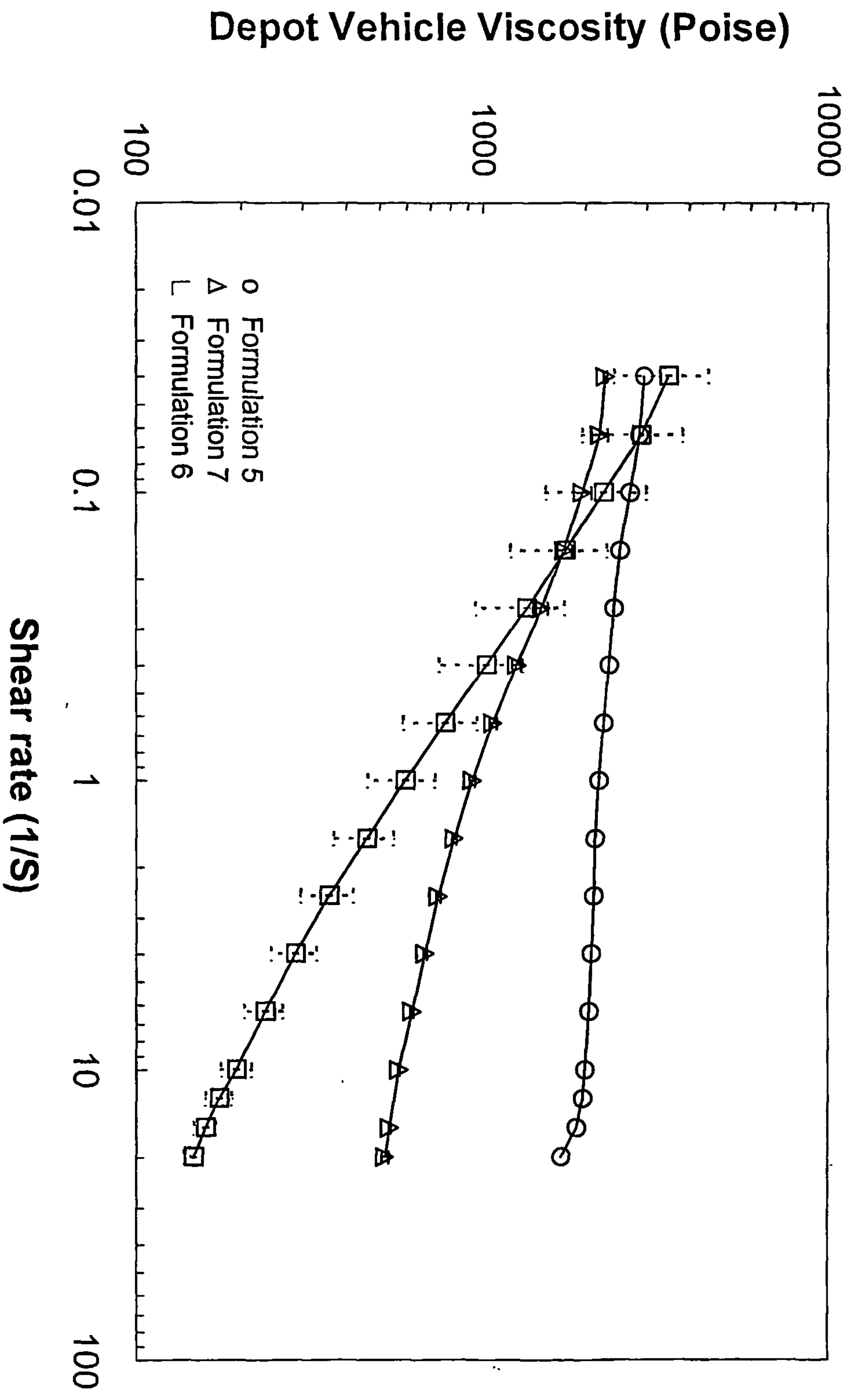

Depot Vehicle Viscosity (Poise)
100
1000
10000
0.01
0.1
1
10
100
Shear rate (1/S)
o Formulation 5
Δ Formulation 7
□ Formulation 6


FIG. 1

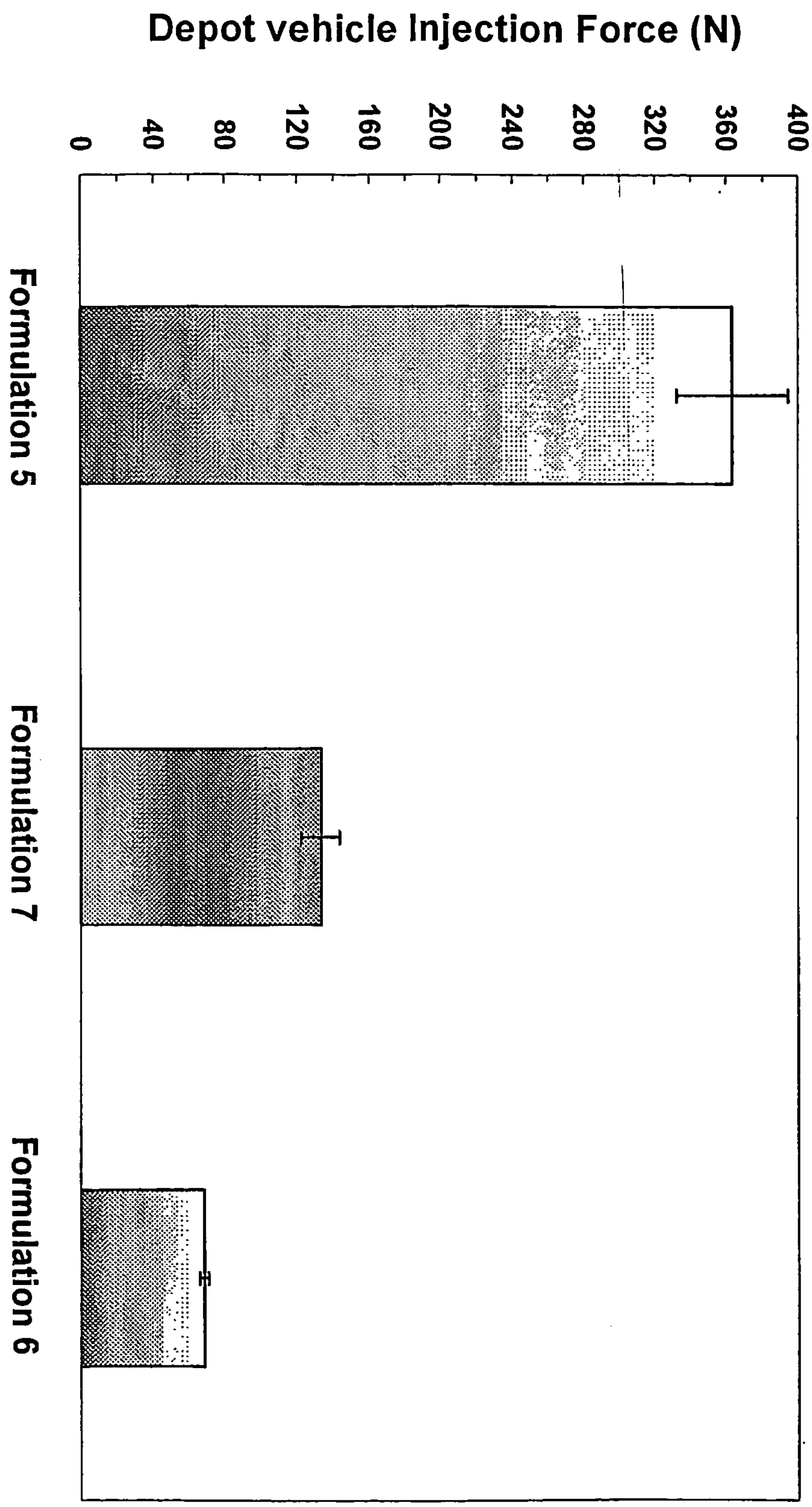
Depot vehicle Injection Force (N)
0
40
80
120
160
200
240
280
320
360
400
Formulation 5
Formulation 7
Formulation 6

FIG. 2

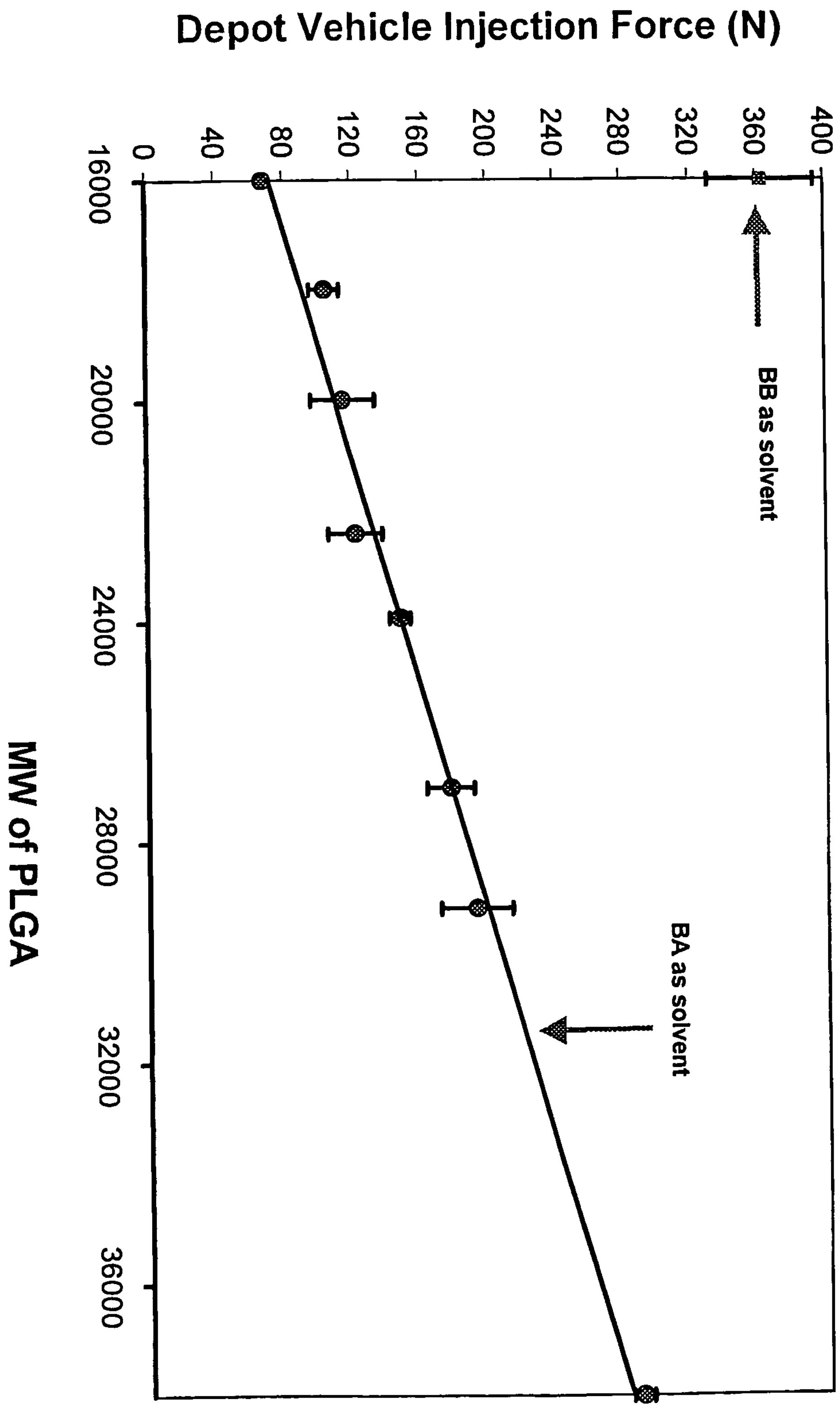
Depot Vehicle Injection Force (N)
0
40
80
120
160
200
240
280
320
360
400
BB as solvent
BA as solvent
16000
20000
24000
28000
32000
36000
MW of PLGA

FIG. 3

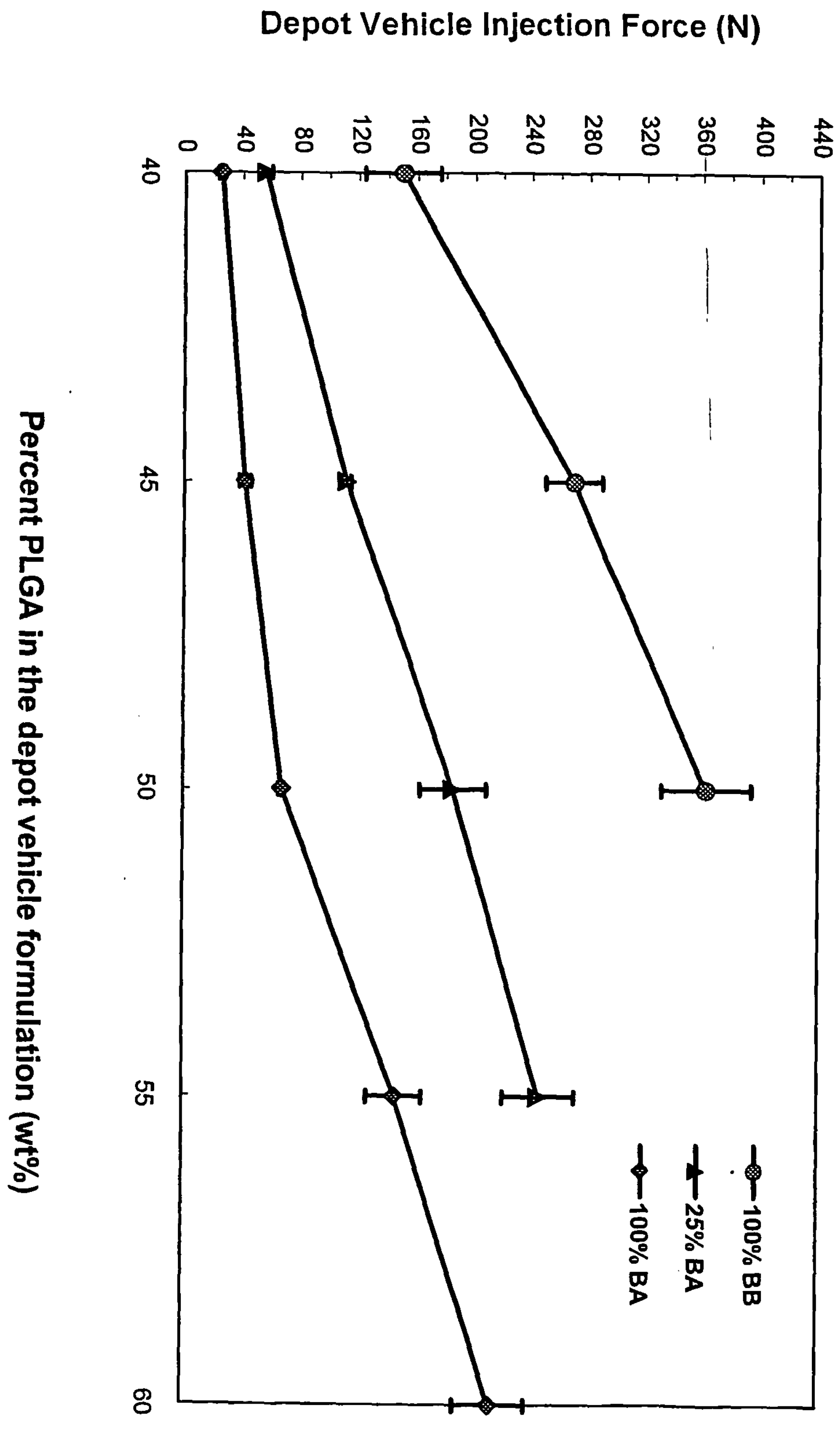
Depot Vehicle Injection Force (N)
0
40
80
120
160
200
240
280
320
360
400
440
40
45
50
55
60
Percent PLGA in the depot vehicle formulation (wt%)
100% BB
25% BA
100% BA

FIG. 5

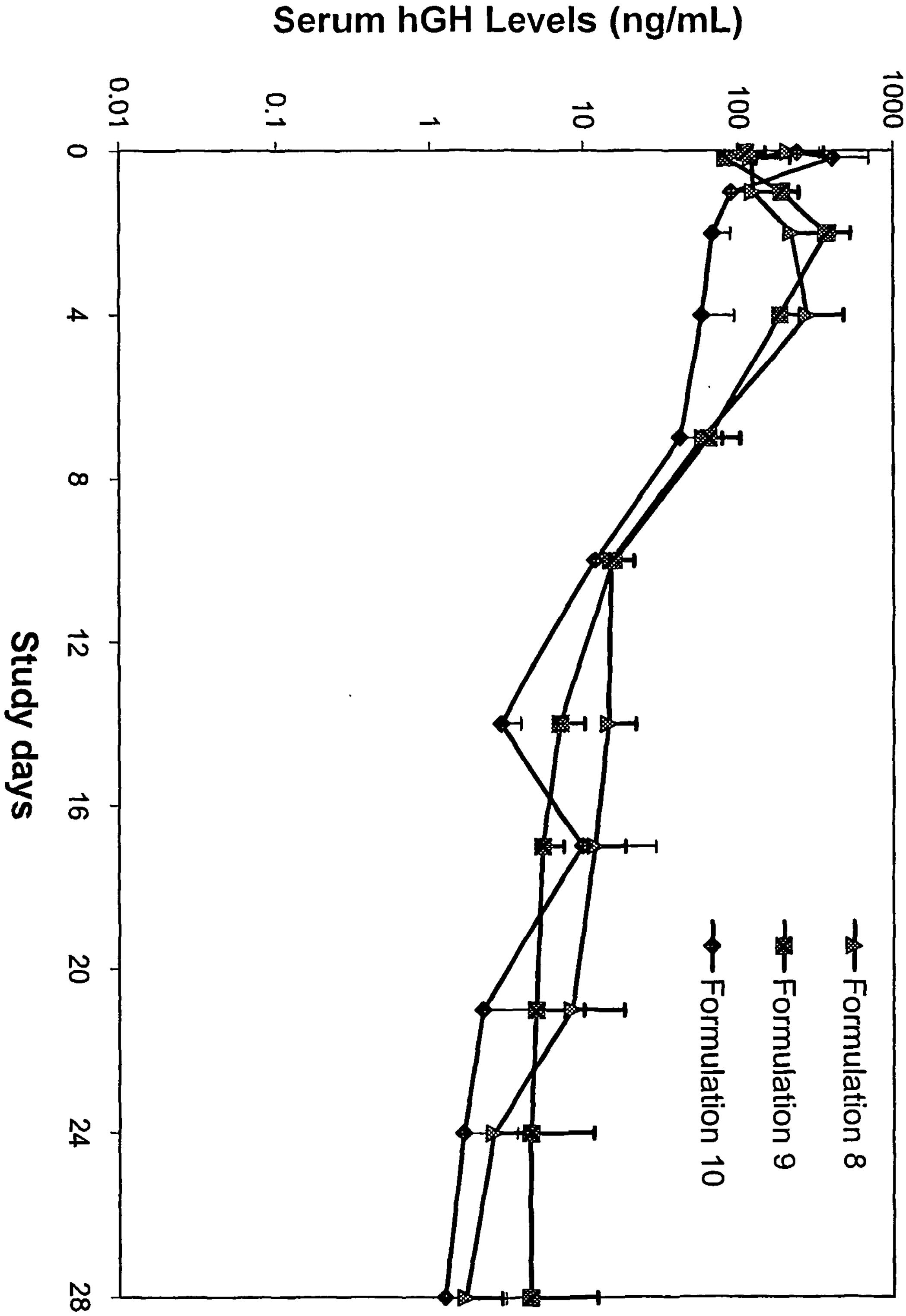
Serum hGH Levels (ng/mL)
1000
100
10
1
0.1
0.01
0
4
8
12
16
20
24
28
Study days
Formulation 8
Formulation 9
Formulation 10

FIG. 6

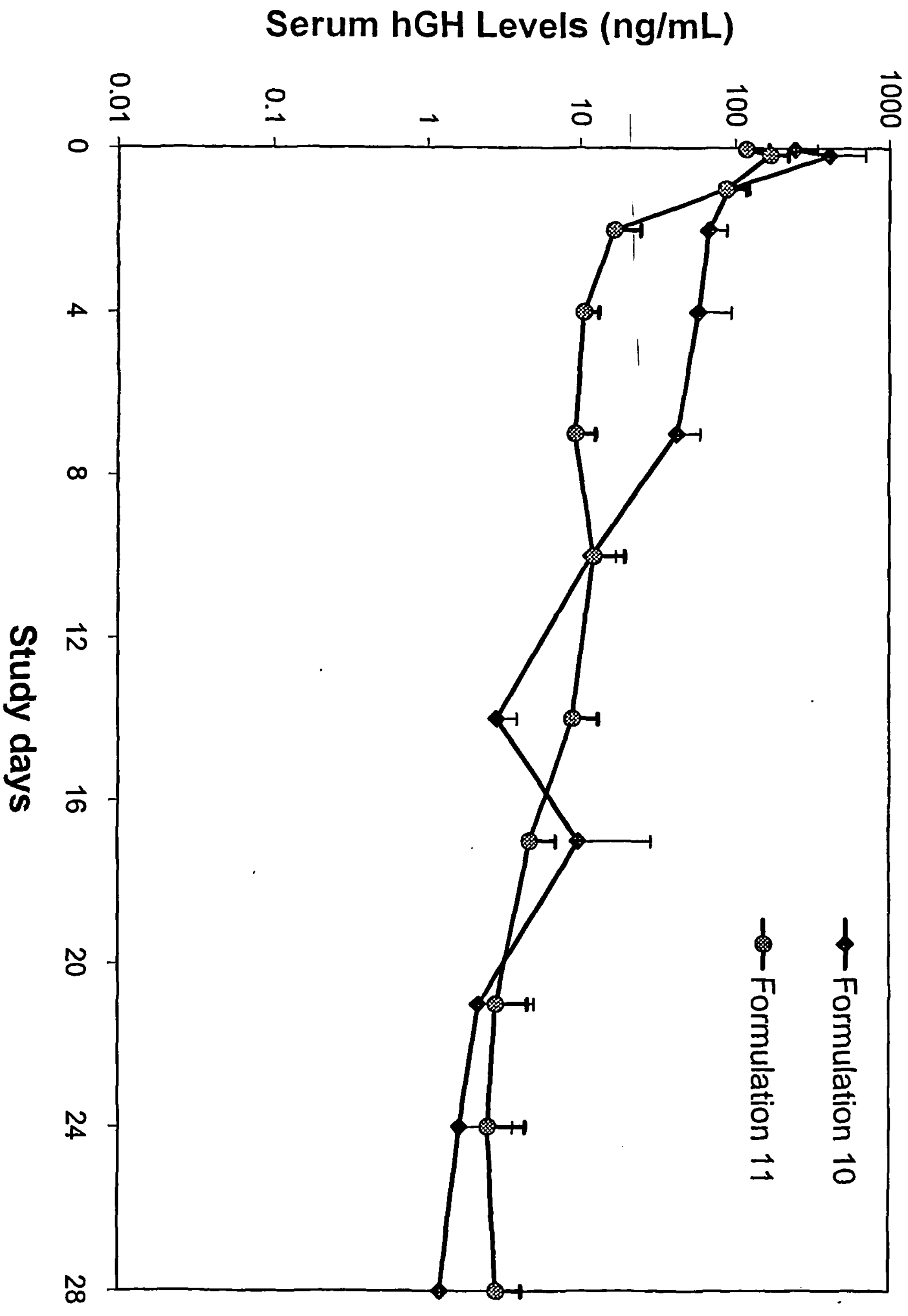
Serum hGH Levels (ng/mL)
1000
100
10
1
0.1
0.01
0
4
8
12
16
20
24
28
Study days
Formulation 10
Formulation 11

FIG. 7

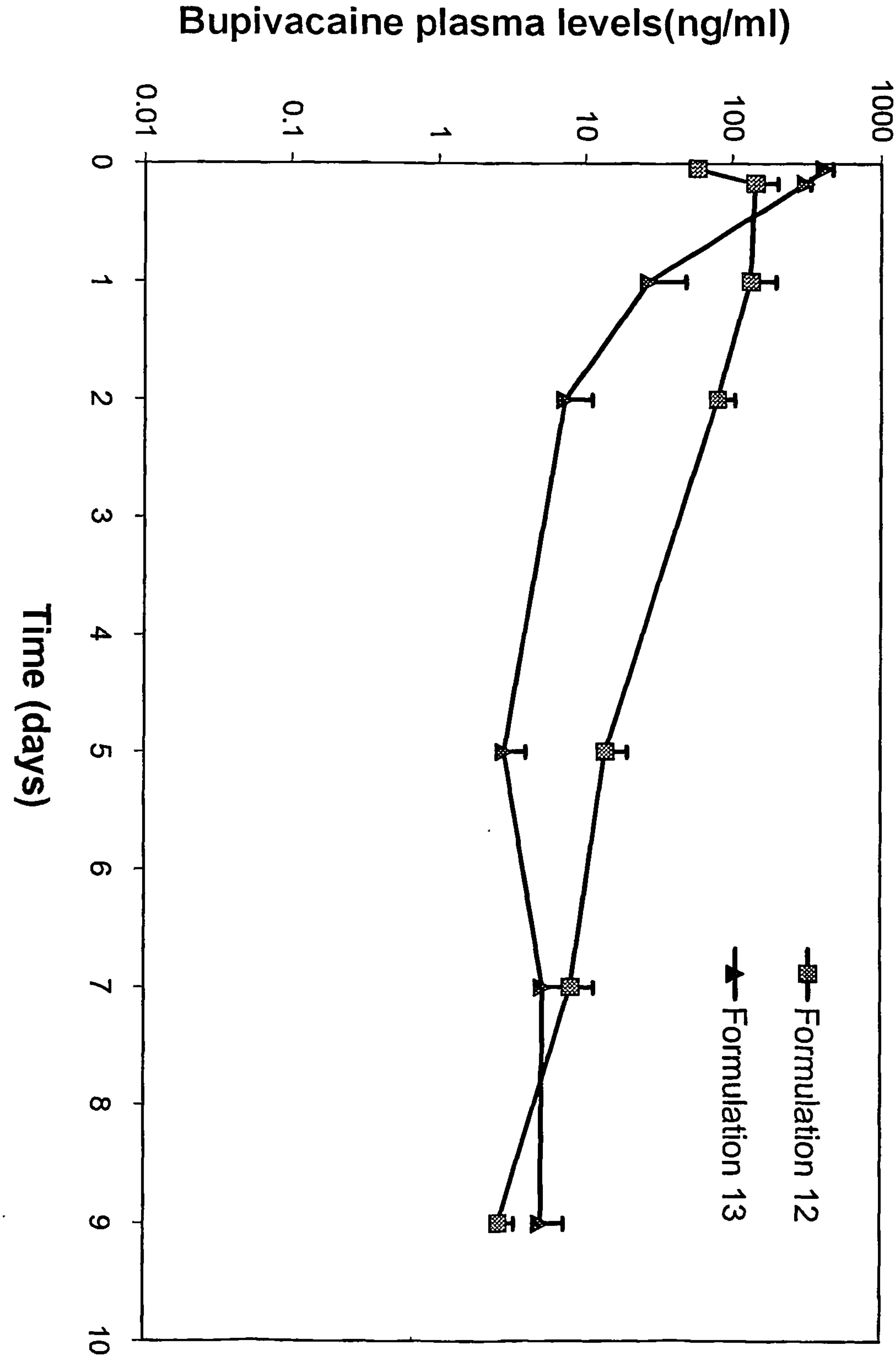
Bupivacaine plasma levels(ng/ml)
1000
100
10
1
0.1
0.01
Time (days)
0
1
2
3
4
5
6
7
8
9
10
Formulation 12
Formulation 13

FIG. 7

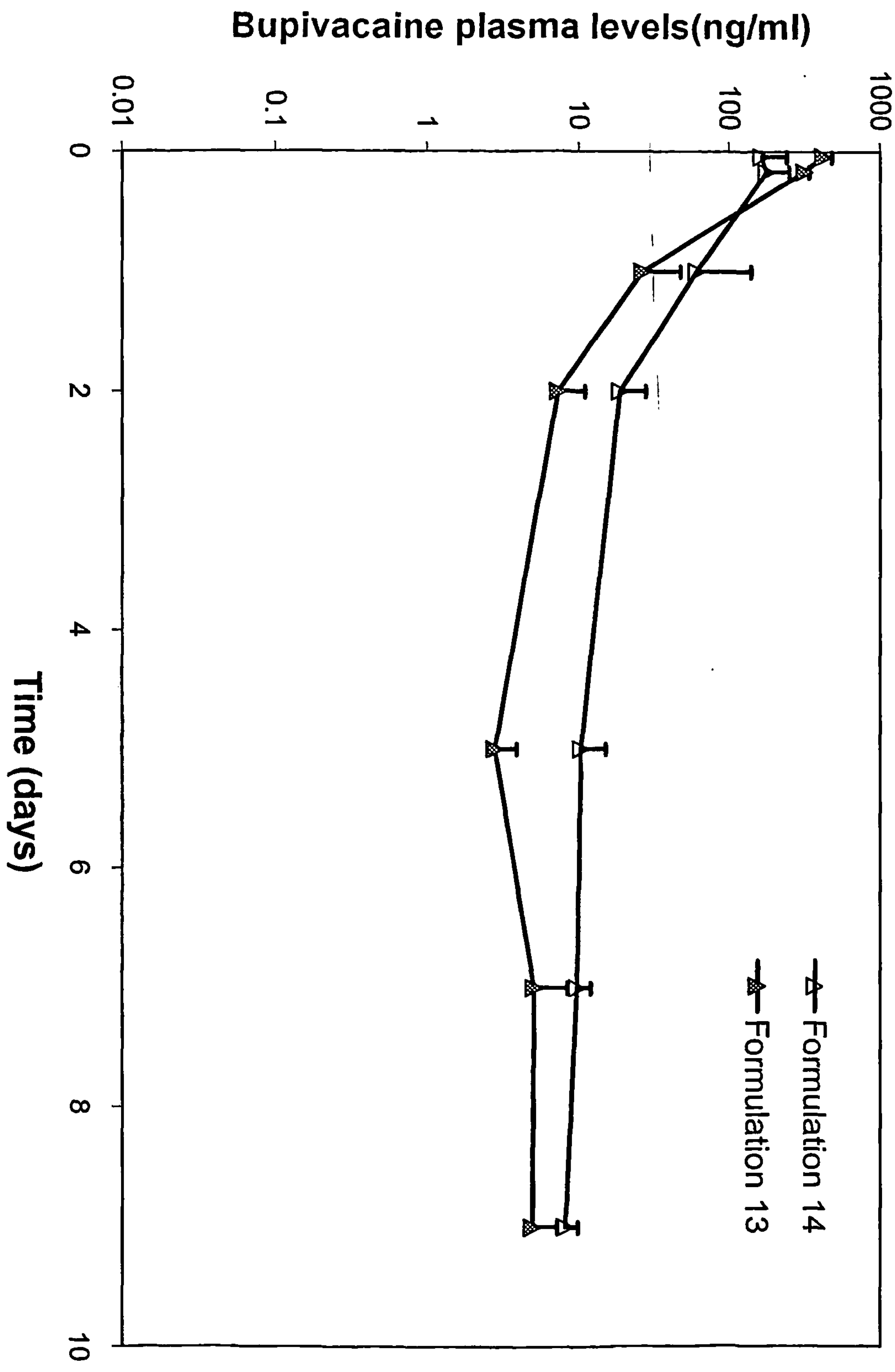
Bupivacaine plasma levels(ng/ml)
1000
100
10
1
0.1
0.01
0
2
4
6
8
10
Time (days)
Formulation 14
Formulation 13

FIG. 8

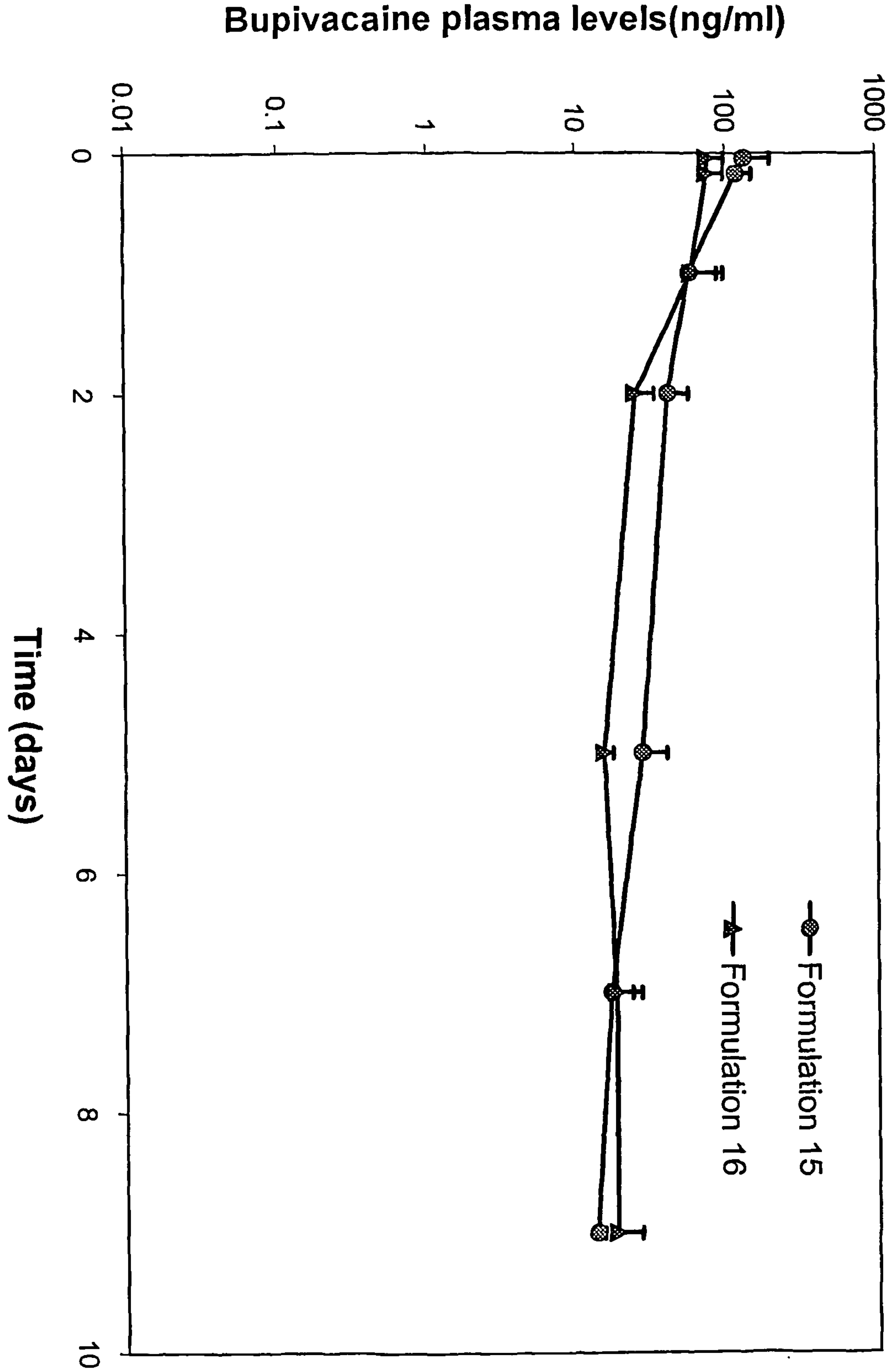
Bupivacaine plasma levels(ng/ml)
1000
100
10
1
0.1
0.01
0
2
4
6
8
10
Time (days)
Formulation 15
Formulation 16

FIG. 9

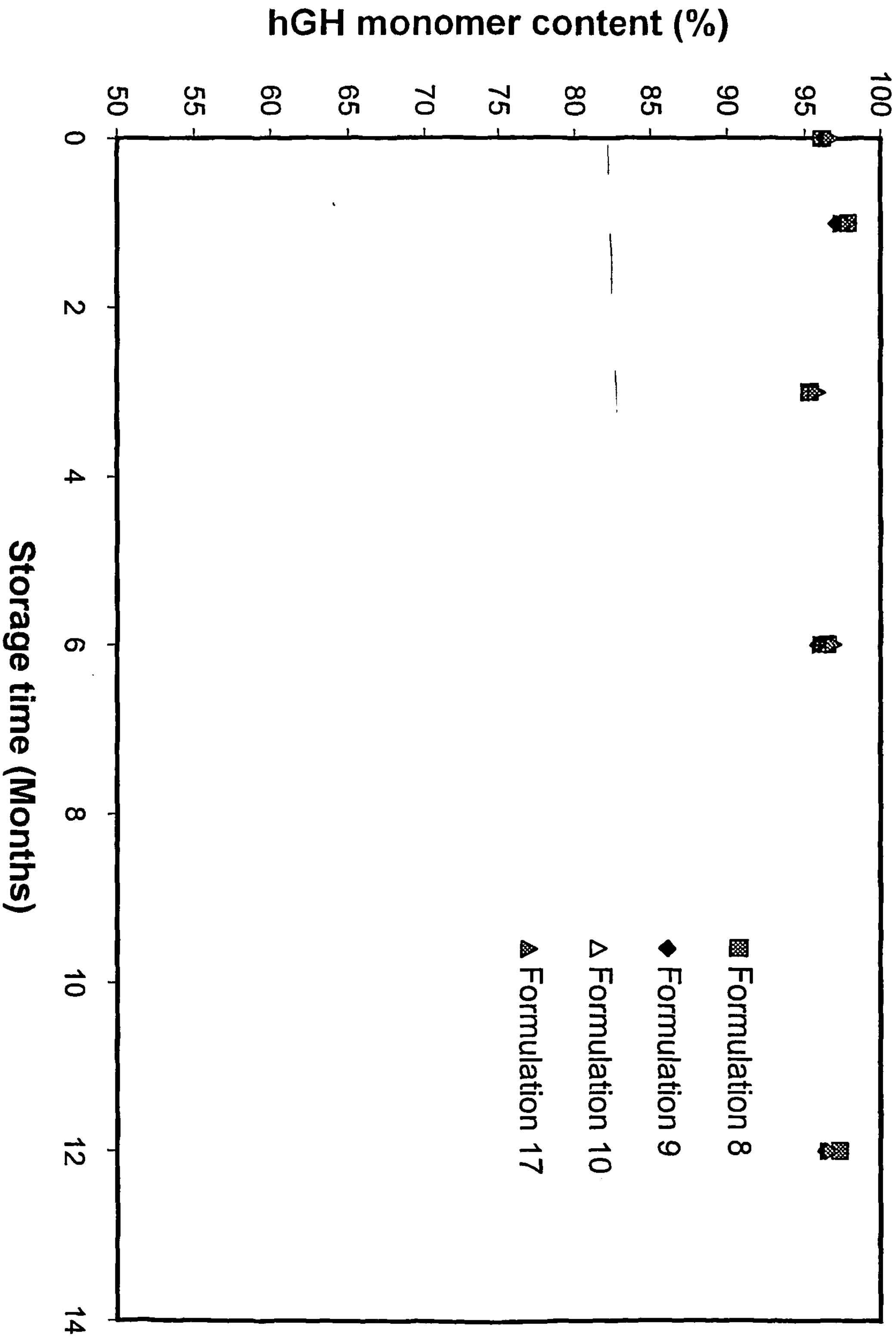
hGH monomer content (%)
50
55
60
65
70
75
80
85
90
95
100
0
2
4
6
8
10
12
14
Storage time (Months)
Formulation 8
Formulation 9
Formulation 10
Formulation 17

FIG. 10

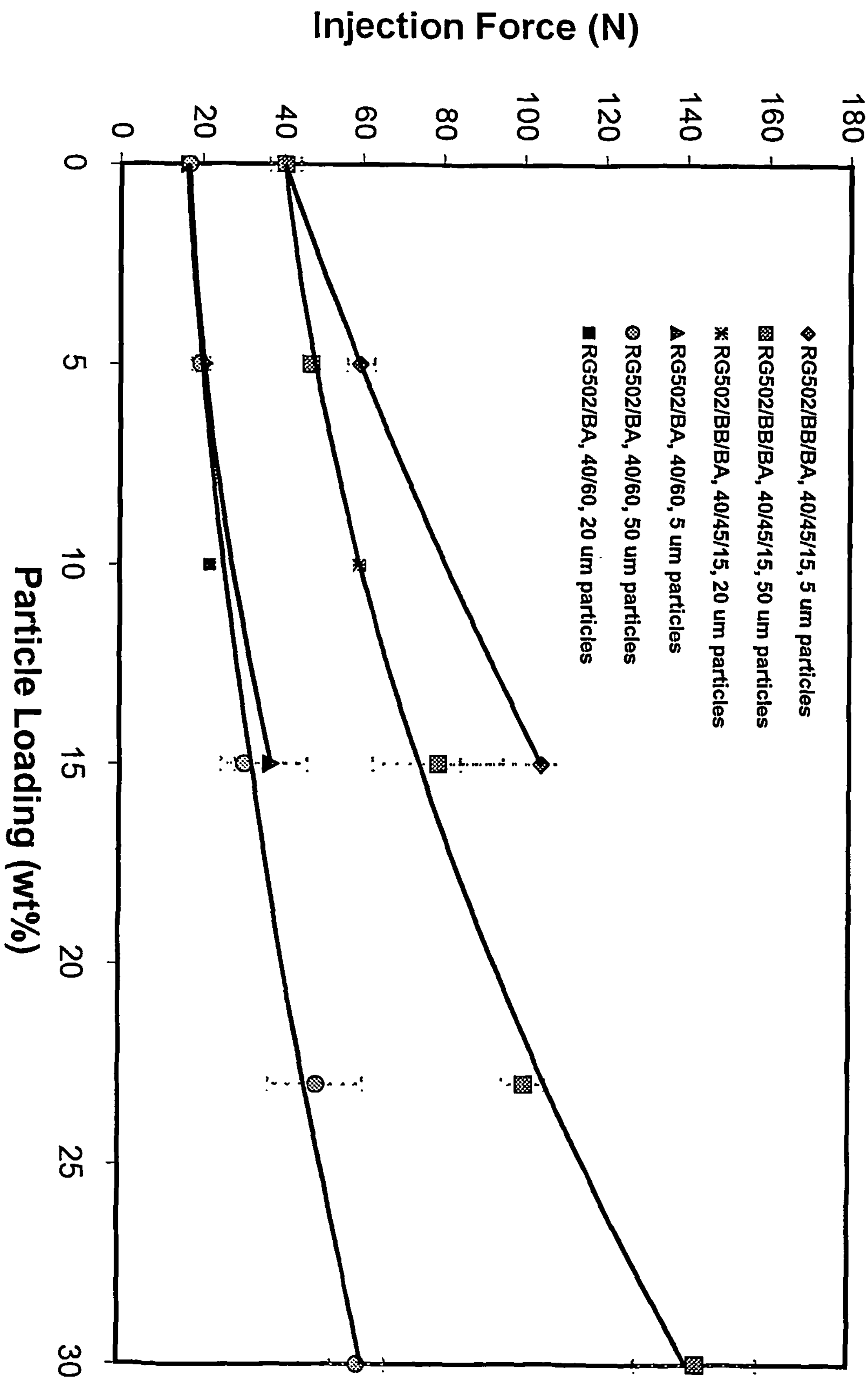
Injection Force (N)
0
20
40
60
80
100
120
140
160
180
RG502/BB/BA, 40/45/15, 5 um particles
RG502/BB/BA, 40/45/15, 50 um particles
RG502/BB/BA, 40/45/15, 20 um particles
RG502/BA, 40/60, 5 um particles
RG502/BA, 40/60, 50 um particles
RG502/BA, 40/60, 20 um particles
0
5
10
15
20
25
30
Particle Loading (wt%)

FIG. 11

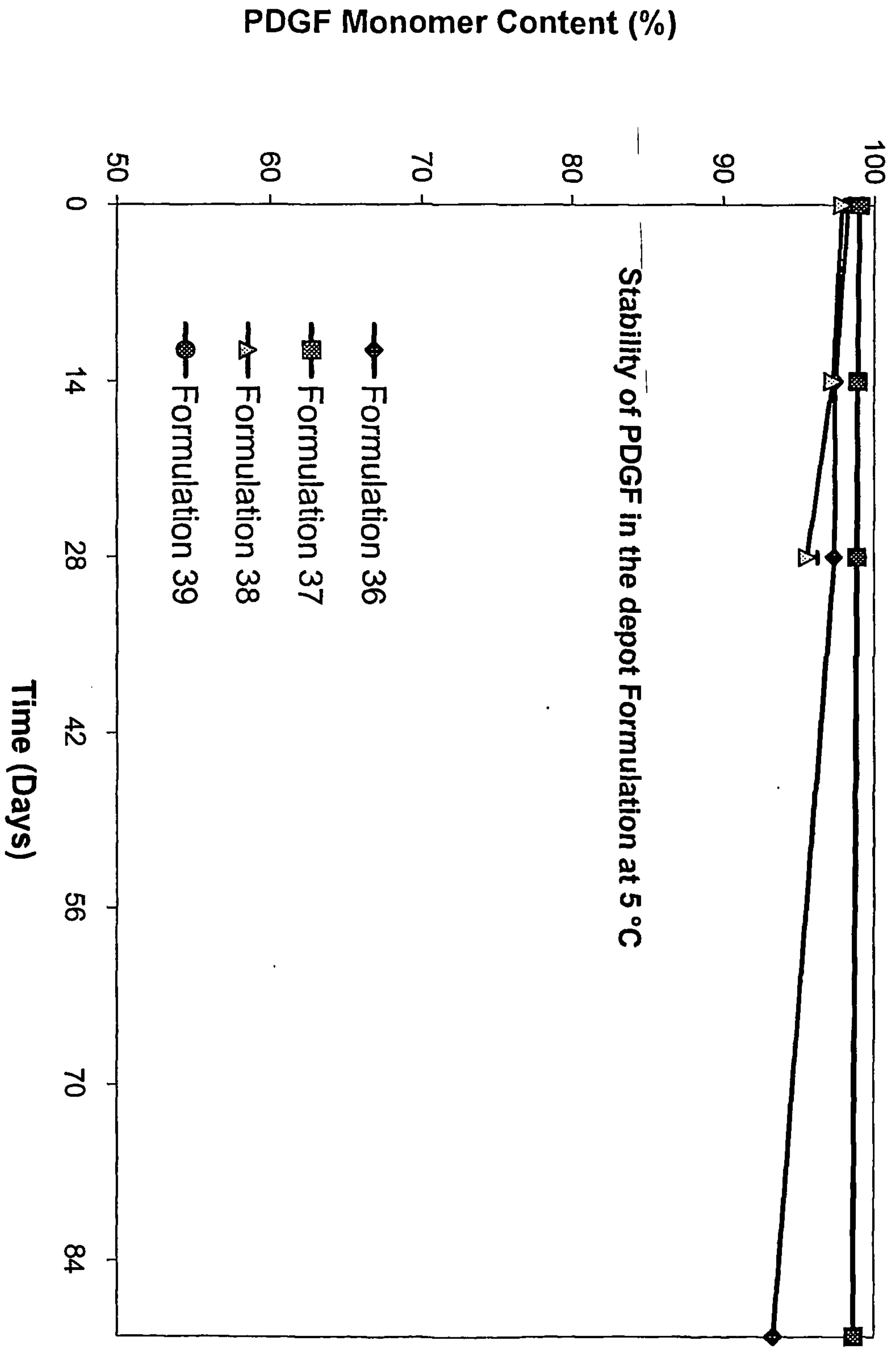
Stability of PDGF in the depot Formulation at 5 °C
PDGF Monomer Content (%)
100
90
80
70
60
50
Time (Days)
0
14
28
42
56
70
84
Formulation 36
Formulation 37
Formulation 38
Formulation 39

FIG. 12

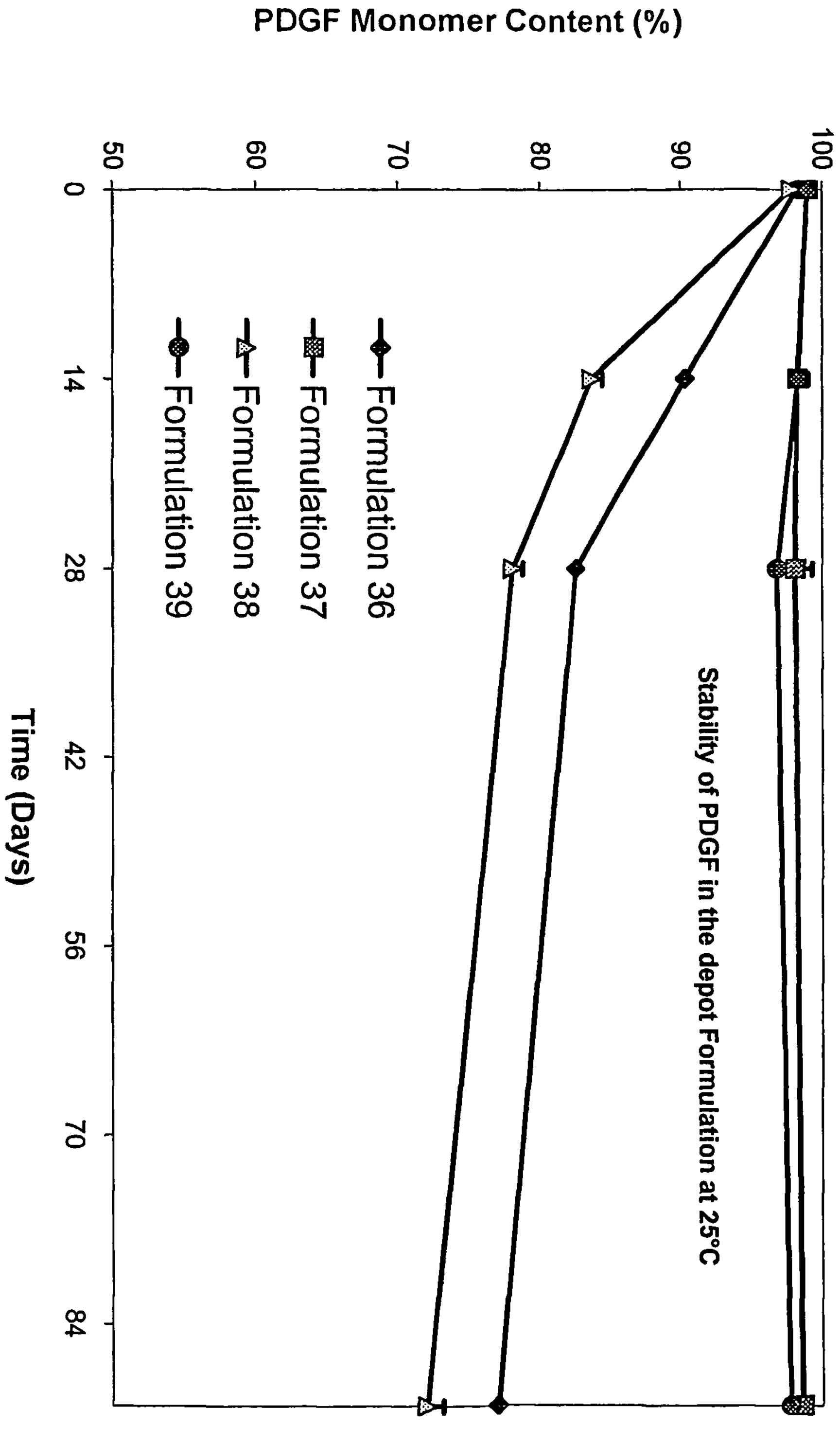
PDGF Monomer Content (%)
100
90
80
70
60
50
Stability of PDGF in the depot Formulation at 25°C
Formulation 36
Formulation 37
Formulation 38
Formulation 39
0
14
28
42
56
70
84
Time (Days)

FIG. 13

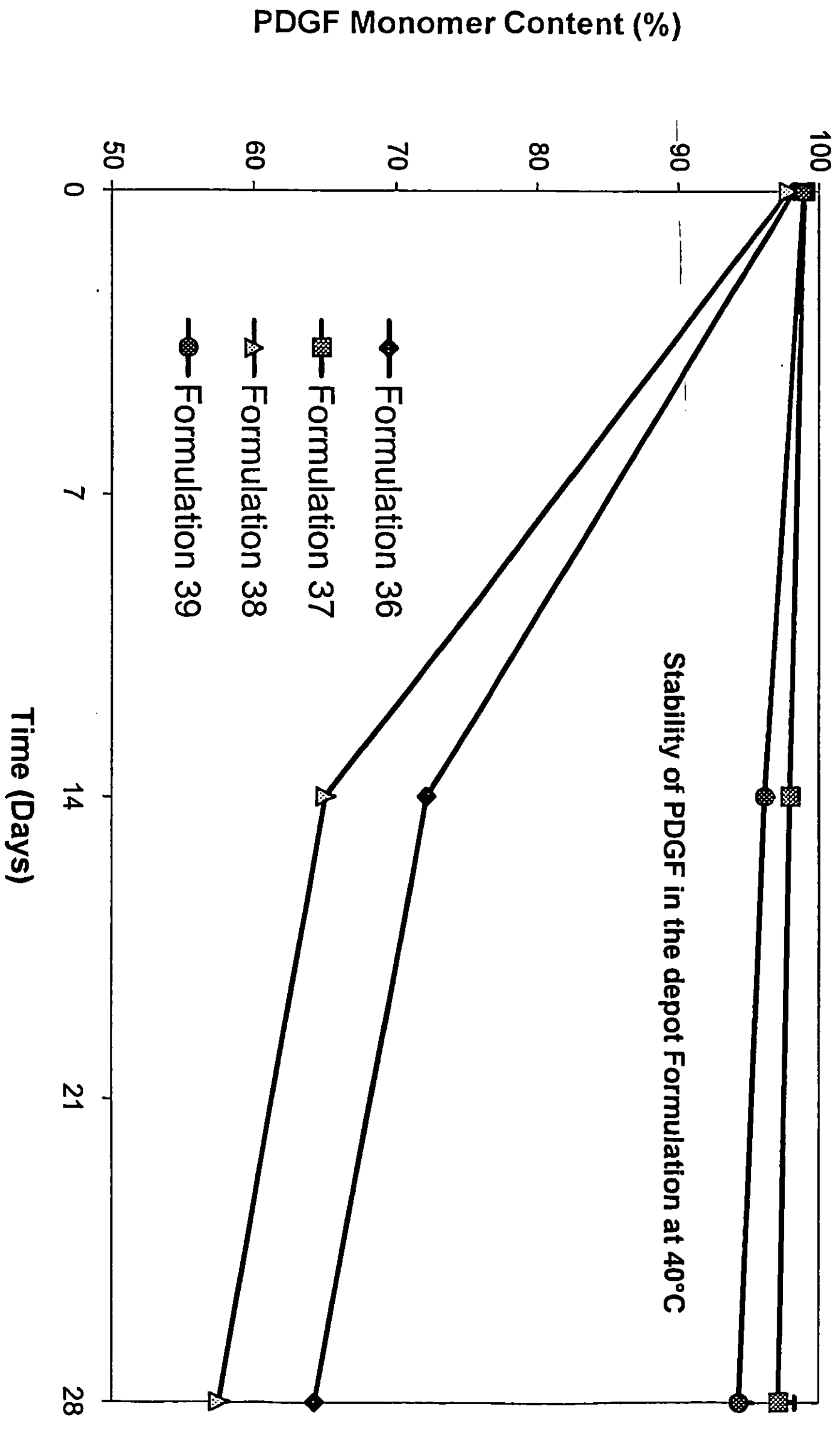
Stability of PDGF in the depot Formulation at 40°C
PDGF Monomer Content (%)
100
90
80
70
60
50
0
7
14
21
28
Time (Days)
Formulation 36
Formulation 37
Formulation 38
Formulation 39

FIG. 14

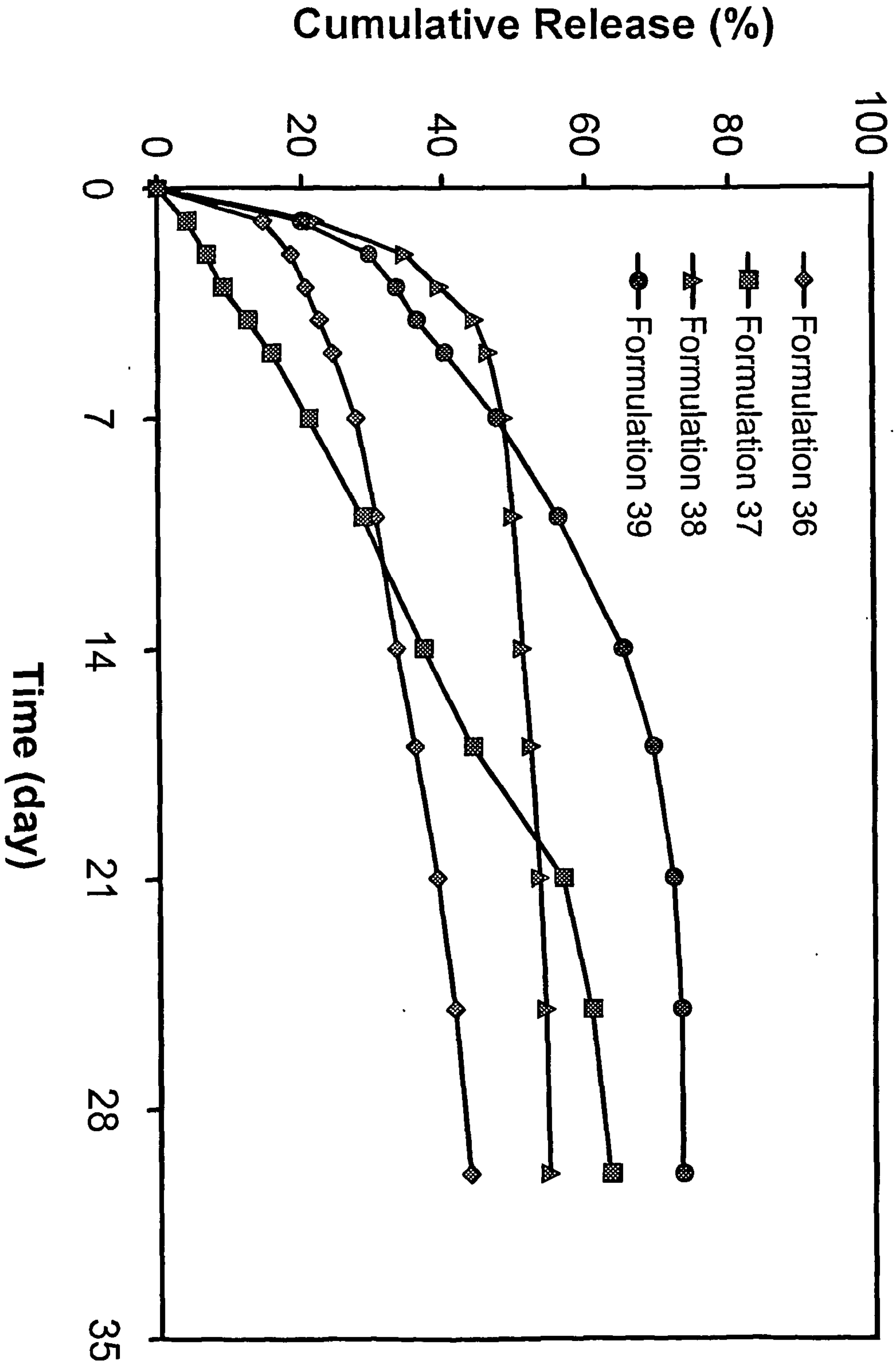
Cumulative Release (%)
0
20
40
60
80
100
Time (day)
0
7
14
21
28
35
Formulation 36
Formulation 37
Formulation 38
Formulation 39

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5085866 A
- US 3797492 A
- US 3987790 A
- US 4008719 A
- US 4865845 A
- US 5057318 A
- US 5059423 A
- US 5112614 A
- US 5137727 A
- US 5151093 A
- US 5234692 A
- US 5234693 A
- US 5279608 A
- US 5336057 A
- US 5209746 A
- US 5308348 A
- US 5456679 A
- US 5019400 A
- US 4938763 A
- US 5278201 A
- US 5599552 A
- US 5242910 A
- US 5620700 A
- US 5556905 A
- US 5656297 A
- US 5654010 A
- US 4985404 A
- US 4853218 A
- US 3923939 A
- US 6130200 A
- WO 9827963 A
- WO 0074650 A
- US 5733950 A
- US 5780044 A
- US 5990194 A, Dunn
- WO 9827962 A
- US 4443340 A
- US 5310865 A
- US 5651986 A